# EUROPEAN PATENT SPECIFICATION

(11) **EP 4 267 951 B1**
(45) Date of publication and mention of the grant of the patent: **25.03.2026**
(21) Application number: 21843660.8
(22) Date of filing: 20.12.2021
(51) Int. Cl.: G01N 27/623

(54) **AUTOMATED CLINICAL DIAGNOSTIC SYSTEM AND METHOD**
SYSTEM UND VERFAHREN ZUR AUTOMATISIERTEN KLINISCHEN DIAGNOSE
SYSTÈME DE DIAGNOSTIC CLINIQUE AUTOMATISÉ ET PROCÉDÉ

(30) Priority: 22.12.2020 EP 20216371
(43) Date of publication of application: 01.11.2023
(60) Divisional application: 26157422.2
(73) Proprietor: F. Hoffmann-La Roche AG, 4070 Basel (CH); Roche Diagnostics GmbH, 68305 Mannheim (DE)
(72) Inventor: EPPING, Claudia, 82377 Penzberg (DE); REMPT, Martin, 82377 Penzberg (DE)
(74) Representative: Epping, Claudia Viktoria
(86) International application number: PCT/EP2021/086819
(87) International publication number: WO 2022/136286

(56) References cited:
- WO-A1-2017/103180
- WO-A1-2020/020850
- WO-A1-2020/079647
- US-A1- 2015 233 866
- US-A1- 2018 033 600
- US-A1- 2019 293 611
- US-A1- 2019 293 612
- US-A1- 2019 293 615
- US-A1- 2020 041 470
- US-A1- 2020 124 576

## Description

### Field of the Invention

The present invention relates to an automated clinical diagnostic system and an automated method for determining the presence or level of at least one analyte of interest in a biological sample.

### Background of the Invention

Mass spectrometry (MS) is a widely used technique for the qualitative and quantitative analysis of chemical substances ranging from small molecules to macromolecules. In general, it is a very sensitive and specific method, allowing even for the analysis of complex biological, for example (e.g.), environmental or clinical samples. However, for several analytes, especially if analysed from complex biological matrices such as serum, sensitivity of the measurement remains an issue.

Often MS is combined with chromatographic techniques, particularly gas and liquid chromatography such as e.g. HPLC. Here, the analysed molecule (analyte) of interest is separated chromatographically and is individually subjected to mass spectrometric analysis (Higashi et al. (2016) J. of Pharmaceutical and Biomedical Analysis 130 p. 181-190). However, this system has inherent problems of analyte - surface contact and the need for stationary phases and liquid solvents. Furthermore the mechanical parts underlies an inherent stress by being moved and this results in the end in a deteriotion of the equipment. Therefore the systems need intensive maintenance due to the mechanical parts which are used. Stationary phases which are being used for e.g. GC or HPLC also need to be replaced by deteriotion or unremovable chemical blockage.

US 2018/033600 A1 describes a mass spectrometry cartridge and methods of use thereof.

US 2015/233866 A1 describes a method and apparatus for improving ion mobility spectrometry by using a fast and spatially wide ion gate based on local RF field barrier opposed to a switching DC field.

WO 2020/079647 A1 describes apparatus, systems, and methods useful for sampling in mass spectrometry systems. In particular, methods which include functionalizing an outer surface of elongate sampling element, for example, a glass or silica rod with a polypeptide that preferentially binds to an analyte.

There is, however, still a need of replacing the HPLC of MS analysis methods in order to overcome the above mentioned disadvantages.

Additionally, mass spectrometry is a versatile tool over all disciplines in science. For environmental analyses, it can determine traces of pollutants, in mineralogy, it can assess chemical compositions and in chemistry, it can reflect pathways of reactions by measuring certain compounds qualitatively as well as quantitatively.

In clinical diagnostics, the versatile tool of mass spectrometry meets different aspects in terms of sample types (e.g. liquid (e.g. serum/blood), solid (e.g. cross sections of tissue) or gaseous (e.g. cross-sections of tissue)) as well as analytical tasks e.g. qualitative or quantitative.

Mass spectrometry in the clinical analysis is further hitting the needs for dealing with low mass analytes e.g. alkali metals (Na, K etc.) or small acids/bases e.g. valproic acid as well as medium size analytes (e.g. steroids) and large molecular mass analytes e.g. biopolymers like proteins. All of the analytes need to be addressed quantitatively as well as qualitatively.

Additionally, there is growing interest for the implementation of mass spectrometry in the clinical laboratory. The number of published methods especially for small molecules in therapeutic drug monitoring or drug of abuse testing is increasing.

Some ready to use kits for pre-validated clinical MS applications are becoming commercially available.

The use of mass spectrometry, however, even in connection with such kits, may not be regulatory approved for clinical diagnostics. This is mostly because of lack of standardized procedures, except for a very few analytes, and because of the still large number of user dependent factors, e.g. due to a number of manual steps that are still conducted and the diversity of hardware components that may be used and combined, and that play a role in delivering reliable and reproducible results of clinical relevance. In particular, sample preparation is typically a manual and tedious procedure. Protein precipitation with subsequent centrifugation is the most popular method to remove unwanted and potentially disturbing sample matrix. The use of kits may in part facilitate sample preparation that can be at least in part automated. Kits are however available only for a limited number of analytes of interest and the entire process from sample preparation, to separation and detection remains complex, requiring the attendance of highly trained laboratory personnel to run highly sophisticated instruments.

Also, typically, a batch approach is followed, where a batch of samples prepared in advance under the same preparation conditions undergo consecutive separation runs under the same separation conditions. This approach however does not enable high throughput and is not flexible, e.g. does not allow re-scheduling (changing a pre-defined processing sequence) in view for example of incoming emergency samples that have higher priority and have to be processed first.

A system and a method are herein described that make use mass spectrometry more convenient and more reliable and therefore suitable for clinical diagnostics. In particular, high-throughput, e.g. up to 100 samples/hour or more with random access for analytes and and sample concentrations/ matrix components sample preparation can be obtained. Moreover the process can be fully automated increasing the walk-away time and decreasing the level of skills required.

There is thus an urgent need in the art for a clinical diagnostic system and a method which allows for a compfortable, low maintainance and fast detection of analytes from complex biological matrices.

Further, the coupling of a sample containing an analyte of interest as decribes herein via an ion generation source to an ion mobility unit, e.g. a structures for lossless ion manipulation unit and an ion detection system can overcome the above-mentioned limitations.

It is an object of the present invention to provide a clinical diagnostic system and a method for determining the presence or level of at least one analyte of interest in a biological sample for efficiently and/or low maintenance and/or fast and/or robust detection of an analyte of interest.

This object is or these objects are solved by the subject matter of the independent claims. Further embodiments are subjected to the dependent claims.

### Summary of the Invention

In an aspect, the present invention relates to an automated clinical diagnostic system comprising the features defined in independent claim 1.

In a further aspect, the present invention relates to an automated method for determining the presence or level of at least one analyte of interest in a biological sample comprising the steps defined in independent claim 12.

### Description of the Figures

**Figures 1 to 8** show a schematic representation of a clinical diagnostic system and a clinical diagnostic method.
**Figure 9** shows schematically elements of a clinical diagnostic method and in particular a combination of possible workflow paths, depending on the sample type and/or the analytes of interest in a sample.
**Figures 10** **and** **11** show an examplified flow chart of the clinical diagnostic method.
**Figure 12A** **and** **12B** show two generic examples of analyte specific workflow paths that may be pre-defined in a master table or memory, each including a selection of options among generally selectable options.

### Detailed Description of the Invention

Before the present invention is described in detail below, it is to be understood that this invention is not limited to the particular embodiments and examples described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Several documents are cited throughout the text of this specification.

In the following, the elements of the present invention will be described. These elements are listed with specific embodiments, however, it should be understood that they may be combined in any manner and in any number to create additional embodiments. The various described examples and preferred embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

### Definitions

The word **"comprise",** and variations such as "comprises" and "comprising", will be understood to imply the inclusion of a stated integer or step or group of integers or steps but not the exclusion of any other integer or step or group of integers or steps. The term "including" and "comprising" can be used interchangeable.

As used in this specification and the appended claims, the singular forms **"a", "an",** and **"the"** include plural referents, unless the content clearly dictates otherwise.

**Percentages, concentrations, amounts, and other numerical data** may be expressed or presented herein in a "range" format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "4% to 20 %" should be interpreted to include not only the explicitly recited values of 4 % to 20 %, but to also include individual values and sub-ranges within the indicated range. Thus, included in this numerical range are individual values such as 4, 5, 6, 7, 8, 9, 10, ... 18, 19, 20 % and sub-ranges such as from 4-10 %, 5-15 %, 10-20%, etc. This same principle applies to ranges reciting minimal or maximal values. Furthermore, such an interpretation should apply regardless of the breadth of the range or the characteristics being described.

The term **"about"** when used in connection with a numerical value is meant to encompass numerical values within a range having a lower limit that is 5% smaller than the indicated numerical value and having an upper limit that is 5% larger than the indicated numerical value.

A **"clinical diagnostics system"** is preferably a laboratory automated apparatus dedicated to the analysis of samples for in vitro diagnostics. The clinical diagnostics system may have different configurations according to the need and/or according to the desired laboratory workflow. Additional configurations may be obtained by coupling a plurality of stations and/or apparatuses and/or modules and/or units together. A "unit" is a work cell, typically smaller in size than the entire clinical diagnostics system, which has a dedicated function. This function can be analytical but can be also pre-analytical or post analytical or it can be an auxiliary function to any of the pre-analytical function, analytical function or post-analytical function. In particular, a unit can be configured to cooperate with one or more other modules or units for carrying out dedicated tasks of a sample processing workflow, e.g. by performing one or more pre-analytical and/or analytical and/or post-analytical steps. The term "unit" and device can be used interchangeably. In particular, the clinical diagnostics system can comprise one or more analytical apparatuses, designed to execute respective workflows that are optimized for certain types of analysis, e.g. clinical chemistry, immunochemistry, coagulation, hematology, liquid chromatography separation, mass spectrometry, etc. Thus the clinical diagnostic system may comprise one analytical apparatus or a combination of any of such analytical apparatuses with respective workflows. Each of the analytical apparatuses may comprise at least one station and/or at least one unit, preferably apre-analytical and/or post analytical unit. The clinical diagnostics system can comprise functional units such as liquid handling units for pipetting and/or pumping and/or mixing of samples and/or reagents and/or system fluids, and also functional units for sorting, storing, transporting, identifying, separating, detecting.

The term **"determining"** the level of the analyte of interest, as used herein refers to the quantification or qualification of the analyte of interest, e.g. to determining or measuring the level of the analyte of interest in the pretreated sample.

In this context **"presence of at least one analyte of interest"** encompass the qualification of the analyte of interest by its absolute value and/or its relative signal value to a internal standard/ other analyte and/ or a limit of analyte concentration which is matched with its conentration.

In this context **"level" or "level value"** encompasses the absolute amount, the relative amount or concentration as well as any value or parameter which correlates thereto or can be derived therefrom.

In the context of the present disclosure, the term **"analyte",** "analyte molecule" , or "analyte(s) of interest" are used interchangeably referring the chemical species to be analysed via mass spectrometry. Chemical species suitable to be analysed via mass spectrometry, i.e. analytes, can be any kind of molecule present in a living organism, include but are not limited to nucleic acid (e.g. DNA, mRNA, miRNA, rRNA etc.), amino acids, peptides, proteins (e.g. cell surface receptor, cytosolic protein etc.), metabolite or hormones (e.g. testosterone, estrogen, estradiol, etc.), fatty acids, lipids, carbohydrates, steroids, ketosteroids, secosteroids (e.g. Vitamin D), molecules characteristic of a certain modification of another molecule (e.g. sugar moieties or phosphoryl residues on proteins, methyl-residues on genomic DNA) or a substance that has been internalized by the organism (e.g. therapeutic drugs, drugs of abuse, toxins, etc.) or a metabolite of such a substance. Such analyte may serve as a biomarker. In the context of present invention, the term "biomarker" refers to a substance within a biological system that is used as an indicator of a biological state of said system.

The term **"are connected to each other",** as used herein can refer or refers to a physical contact by either a stationary unit e.g. tube connection or a semi physical connection by. e.g. a pipetting unit. Furthermore physical can mean a contact in solid/liquid (e.g. aqustic droplet ejection or piezo driven droplet ejection) or gaseous (e.g. ion optic with an ion mirror, an S-lense or a multipole).

The term **"directly",** as used herein may mean that no other stations are arranged between the two directly connected stations.

Analytes may be present in a sample of interest, e.g. a biological sample, preferably a clinical biological sample. The term **"sample"** or "sample of interest" or "biological sample" are used interchangeably herein, referring to a part or piece of a tissue, organ or individual, typically being smaller than such tissue, organ or individual, intended to represent the whole of the tissue, organ or individual. Upon analysis a sample provides information about the tissue status or the health or diseased status of an organ or individual. Examples of samples include but are not limited to fluid samples such as blood, serum, plasma, synovial fluid, spinal fluid, urine, saliva, and lymphatic fluid, or solid samples such as dried blood spots and tissue extracts. Further examples of samples are cell cultures or tissue cultures.

The term **"serum"** as used herein is the clear liquid part of the blood hat can be separated from clotted blood. The term **"plasma"** as used herein is the clear liquid part of blood which contains the blood cells. Serum differs from plasma, the liquid portion of normal unclotted blood containing the red and white cells and platelets. It is the clot that makes the difference between serum and plasma. The term **"whole blood"** as used herein contains all components of blood, for examples white and red blood cells, platelets, and plasma.

In the context of the present disclosure, the sample may be derived from an **"individual"** or "subject" or "patient". Typically, the subject or patient is a mammal. Mammals include, but are not limited to, domesticated animals (e.g., cows, sheep, cats, dogs, and horses), primates (e.g., humans and non-human primates such as monkeys), rabbits, and rodents (e.g., mice and rats). Preferably, the patient smaple is from a human.

The term **"small molecule"** as used herein is a molecule or compound having a molecular weight or molar mass of less than 2000 Da preferably less than 1000 Da, more preferably 800 Da.

A **"sample preparation station"** is a pre-analytical module coupled to one or more analytical stations or units designed to execute a series of sample processing steps aimed at removing or at least reducing interfering matrix components in a sample and/or enriching analytes of interest in a sample. Such processing steps may include any one or more of the following processing operations carried out on a sample or a plurality of samples, sequentially, in parallel or in a staggered manner: pipetting (aspirating and/or dispensing) fluids, pumping fluids, mixing with reagents, incubating at a certain temperature, heating or cooling, centrifuging, separating, filtering, sieving, drying, washing, resuspending, aliquoting, transferring, storing...).

A **"reagent"** is a substance used for treatment of a sample in order e.g. to prepare a sample for analysis, to enable a reaction to occur, or to enable detection of a physical parameter of the sample or analyte contained in the sample. In particular, a reagent can be a substance that is or comprises a reactant, typically a compound or agent capable e.g. of binding to or chemically transforming one or more analytes present in a sample or an unwanted matrix component of the sample. Examples of reactants are enzymes, enzyme substrates, conjugated dyes, protein-binding molecules, ligands, nucleic acid binding molecules, antibodies, chelating agents, promoters, inhibitors, epitopes, antigens, and the like. However the term reagent is used to include any fluid that can be added to a sample including a dilution liquid, including water or other solvent or a buffer solution, or a substance that is used for disruption of specific or nonspecific binding of an analyte to a protein, binding proteins or surfaces.

Sample may be provided for example in sample containers such as sample tubes, including primary tubes and secondary tubes, or multi-well plates, or any other sample carrying support. Reagents may be arranged for example in the form of containers or cassettes containing individual reagents or group of reagents and placed in appropriate receptacles or positions within a storage compartment or conveyor. Other types of reagents or system fluids may be provided in bulk containers or via a line supply.

The term **"automatically" or "automated"** as used herein is a broad term and is to be given its ordinary and customary meaning to a person of ordinary skill in the art and is not to be limited to a special or customized meaning. The term specifically may refer, without limitation, to a process which is performed completely by means of at least one computer and/or computer network and/or machine, in particular without manual action and/or interaction with a user.

The term **"fully automated"** may refer to a process which is performed completely by means of at least one computer and/or computer network and/or machine, without manual action and/or interaction with a user.

The term **"partially automated"** may refer to a process which is performed by means of at least one computer and/or computer network and/or machine and with the aid of manual action and/or interaction with a user. Preferably, "partially automated" can mean that the manual action and/or interaction with a user is 50% or 40% or 30% or 20% or 10% or 5% at the maximum of the total process, wherein the rest of the process is performed by means of at least one computer and/or computer network and/or machine. "By means of at least one computer and/or computer network and/or machine" can mean that this process is performed without any manual action and/or interaction with a user.

Typically, an **"internal standard"** (ISTD) is a known amount of a substance which exhibits similar properties as the analyte of interest when subjected to the mass spectrometric detection workflow (i.e. including any pre-treatment, enrichment and actual detection step). Although the ISTD exhibits similar properties as the analyte of interest, it is still clearly distinguishable from the analyte of interest. Exemplified, during ion mobility separation, the ISTD has the same ion size but different m/z ratio than the analyte of interest. Preferably the internal standard is in its ion size not distinguishable from the analyte but different in the m/z ratio. Thus, both the analyte and the ISTD enter the mass spectrometer at the same time. The ISTD however, exhibits a different molecular mass than the analyte of interest from the sample. This allows a mass spectrometric distinction between ions from the ISTD and ions from the analyte by means of their different mass/charge (m/z) ratios. Both are subject to fragmentation and provide daughter ions. These daughter ions can be distinguished by means of their m/z ratios from each other and from the respective parent ions. Consequently, a separate determination and quantification of the signals from the ISTD and the analyte can be performed. Since the ISTD has been added in known amounts, the signal intensity of the analyte from the sample can be attributed to a specific quantitative amount of the analyte. Thus, the addition of an ISTD allows for a relative comparison of the amount of analyte detected, and enables unambiguous identification and quantification of the analyte(s) of interest present in the sample when the analyte(s) reach the mass spectrometer. Typically, but not necessarily, the ISTD is an isotopically labeled variant (comprising e.g. ²H, ¹³C, or ¹⁵N etc. label) of the analyte of interest.

In the context of the present invention, the term **"derivatisation reagent"** or **"label"** or **"derivatisation agent"** are used interchangeably and refer to a chemical substance having a specific chemical structure. Said compound may comprise one or more reactive groups. Each reactive group may fulfil a different functionality, or two or more reactive groups may fulfil the same funtion. Reactive groups include but are not limited to reactive units, charged units, and neutral loss units. The derivatization reagent can undergo an atmospheric pressure chemical reaction with the analyte of interest.

The term **"hemolysis reagent"** (HR) refers to reagents which lyse cells present in a sample, in the context of this invention hemolysis reagents in particular refer to reagents which lyse the cell present in a blood sample including but not limited to the erythrocytes present in whole blood samples. A well known hemolysis reagent is water (H₂O). Further examples of hemolysis reagents include but are not limited to deionized water, liquids with high osmolarity (e.g. 8M urea), ionic liquids, and different detergents.

In the context of the present invention, the term **"compound"** or **"derivatisation reagent"** or **"label"** or **"derivatisation agent"** are used interchangeably and refer to a chemical substance having a specific chemical structure. Said compound may comprise one or more reactive groups Q. Each reactive group may fulfil a different functionality, or two or more reactive groups may fulfil the same funtion. Reactive groups include but are not limited to reactive units, charged units, and neutral loss units. The derivatization reagent can undergo a chemical reaction with the analyte of interest.

The term **"solid-phase extraction (SPE)"** refers to the adsorption of analytes from the a matrix, e.g. a biological matrix onto a solid sorbent, and subsequent elution of the analytes from the sorbent into an solvent, e.g. an organic solvent. SPE is an efficient tool for the sample preparation in chemistry. Conventional SPE materials are silica-based, carbon-based, and clay-based resins. Many adsorbents for SPE applications are commercially available in different formats in the market such as SPE tubes and pipette tip formats such as Oasis-HLB (produced by Waters), Omix (produced by Agilent), and MonoTips (produced by GL Sciences).

The basic principles of SPE are similar to liquid-liquid extraction. Both methods involve the distribution of dissolved species between two phases. However, SPE involves the dispersion of the analyte between a liquid (sample medium) and a solid (adsorbent) phase instead of the two liquid phases which cannot be not mixed together as in liquid-liquid extraction. This technique allows the enrichment and purification of the analytes on a solid adsorbent through adsorption from the solution. This technique is known for a skilled person and thus not explained in more detail.

The term **"liquid-liquid extraction (LLE)"** refers to a separation process consisting of the transfer of a solute from one solvent to another, the two solvents being immiscible or partially miscible with each other. Frequently, one of the solvents can be water or an aqueous mixture and the other can be a nonpolar organic liquid. As in all extraction processes, liquid-liquid extraction comprises a step of mixing (contacting), followed by a step of phase separation. It is important to consider both steps in the selection of solvents and modes of operation. Thus, while vigorous mixing is favorable to the transfer of the analyte of interest from one solvent to the other, it may also impair the ease of phase separation by forming emulsions. This technique is known for a skilled person and thus not explained in detail.

The term **"chromatography"** refers to a process in which a chemical mixture carried by a liquid or gas is separated into components as a result of differential distribution of the chemical entities as they flow around or over a stationary liquid or solid phase.

The term **"liquid chromatography"** or "LC" refers to a process of selective retardation of one or more components of a fluid solution as the fluid uniformly percolates through a column of a finely divided substance, or through capillary passageways. The retardation results from the distribution of the components of the mixture between one or more stationary phases and the bulk fluid, (i.e., mobile phase), as this fluid moves relative to the stationary phase(s). Methods in which the stationary phase is more polar than the mobile phase (e.g., toluene as the mobile phase, silica as the stationary phase) are termed normal phase liquid chromatography (NPLC) and methods in which the stationary phase is less polar than the mobile phase (e.g., water-methanol mixture as the mobile phase and C18 (octadecylsilyl) as the stationary phase) is termed reversed phase liquid chromatography (RPLC).

**"High performance liquid chromatography"** or "HPLC" refers to a method of liquid chromatography in which the degree of separation is increased by forcing the mobile phase under pressure through a stationary phase, typically a densely packed column. Typically, the column is packed with a stationary phase composed of irregularly or spherically shaped particles, a porous monolithic layer, or a porous membrane. HPLC is historically divided into two different subclasses based on the polarity of the mobile and stationary phases. Methods in which the stationary phase is more polar than the mobile phase (e.g., toluene as the mobile phase, silica as the stationary phase) are termed normal phase liquid chromatography (NPLC) and the opposite (e.g., water-methanol mixture as the mobile phase and C18 (octadecylsilyl) as the stationary phase) is termed reversed phase liquid chromatography (RPLC). Micro LC refers to a HPLC method using a column having a norrow inner column diameter, typically below 1 mm, e.g. about 0.5 mm. "Ultra high performance liquid chromatography" or "UHPLC" refers to a HPLC method using a pressure of 120 MPa (17,405 lbf/in2), or about 1200 atmospheres. Rapid LC refers to an LC method using a column having an inner diameter as mentioned above, with a short length <2 cm, e.g. 1 cm, applying a flow rate as mentioned above and with a pressure as mentioned above (Micro LC, UHPLC). The short Rapid LC protocol includes a trapping / wash / elution step using a single analytical column and realizes LC in a very short time <1 min.

Further well-known LC modi include "hydrophilic interaction chromatography" (HILIC), size-exclusion LC, ion exchange LC, and affinity LC.

LC separation may be single-channel LC or multi-channel LC comprising a plurality of LC channels arranged in parallel. In LC analytes may be separated according to their polarity or log P value, size or affinity, as generally known to the skilled person.

The term **"micro particle"** may refer to spheric particles or unregularly shaped particles in a range of 10 nm-10 mm. The particles can consist of or comprise inorganic and-or organic material e.g. SiO2, TiO2, Polystyrene, PMMA and optionally many more materials. The surface of the particles can be modified by a different chemistry from the supporting microparticle core material e.g. C18 chain, CN, Pentaflurophenyl etc.

The term **"bead"** does not necessarily refer to a spherical shape but to a particle having an average size in the nanometer or micrometer range and having any possible shape. A bead may be a solid phase. Suitable solid phases include but are not limited to Solid Phase Extraction (SPE) cartridges. Beads may be non-magnetic, magnetic, or paramagnetic. Beads may be coated differently to be specific for the analyte of interest. The coating may differ depending on the use intended, i.e. on the intended capture molecule. It is well-known to the skilled person which coating is suitable for which analyte. The beads may be made of various different materials. The beads may have various sizes and comprise a surface with or without pores.

Non-magnetic beads may also be used. In that case capturing and releasing may be based on filtration. The sample preparation station may further comprise one or more pipetting device or fluid transport device for adding/removing fluids, such as samples, reagents, wash fluids, suspension fluids, into/from the reaction container(s).

The term **"inert gas stripping"** may refer to the process of bubbeling an inert gas e.g. nitrogen or argon through a liquid which needs to be stripped. Volatile compounds gets evaporated due to the equilibrium process of the inert gas with the analyte supported liquid.

The term **"headspace extraction"** may refer to the process of having a liquid sample seald gastight in a vial which has air or other gases in the headspace over the liquid. The sealing of the vial is at ambigent pressure. The seald vial is getting heated by external heat source and the analyte of interest evaporats in the headspace while increasing the pressure of the vial. The gaseous portion of the vial if further taken of by a suringe or capillary and directly transfered to the analytical unit.

The term **"gas adsorption supported by liquid and/or solid"** may refer to a process in which a gas is adsorb on the active surface of a liquid or a solid. The analyte can undergo during this process endothermal or exothermal processes as well as chemical reactions.

The term **"unsteady analyte ion supply unit"** may refer to a unit which produces ions time dependend e.g. by a laser shot which is pulsed.

The term **"steady analyte ion supply unit"** may refer to a unit which produces ions time independend e.g. by a constant low and power supply during nano-ESI.

The term **"electrospray ionization"** or "ESI," refers to methods in which a solution is passed along a short length of capillary tube, to the end of which is applied a high positive or negative electric potential. Solution reaching the end of the tube is vaporized (nebulized) into a jet or spray of very small droplets of solution in solvent vapor. This mist of droplets flows through an evaporation chamber, which is heated slightly to prevent condensation and to evaporate solvent. As the droplets get smaller the electrical surface charge density increases until such time that the natural repulsion between like charges causes ions as well as neutral molecules to be released.

The term **"nano electrospray ionization"** or **"nanoESI"** refers to methods typically using flow rates below 1 µL/min either in static or dynamic mode. Preferably, nanoESI uses a flow rate of 50 to 500 nl/min, e.g. 500 nl/min. 500 nl/min is equal to 0.5 µl/min.

The term **"static nanoESI"** is used in the context of the present disclosure as a non-continuous flow nanoESI option. The analysis is typically defined by a discrete sample being loaded by single-use pipette tips into an emitter. In contrast, dynamic nanoESI mass spectrometry is characterized by a mobile phase pumped at low flow rates through a small diameter emitter.

The term **"atmospheric pressure chemical ionization"** or "APCI," refers to mass spectrometry methods that are similar to ESI; however, APCI produces ions by ion-molecule reactions that occur within a plasma at atmospheric pressure. The plasma is maintained by an electric discharge between the spray capillary and a counter electrode. Then ions are typically extracted into the mass analyser by use of a set of differentially pumped skimmer stages. A counterflow of dry and preheated nitrogen gas may be used to improve removal of solvent. The gas-phase ionization in APCI can be more effective than ESI for analysing less-polar entity.

**"High-field asymmetric-waveform ion-mobility spectrometry (FAIMS)"** is an atmospheric pressure ion mobility technique that separates gas-phase ions by their behavior in strong and weak electric fields.

The term "Nano Spray" or "APCI," refers to two independend ionization techniques in which a high electrical current is being used for the ionization.

The term "is arranged before" can refer to the arrangement of the two units, which means that the at least one analyte of interest passes at first the first unit (e.g. first ion fragmentation), which is arranged before the second unit (e.g. ion mobility unit), and then passes the second unit.

The term "is arranged after" can refer to the arrangement of the two units, that two units are arranged after one another. This can mean that the at least one analyte of interest passes at first the first unit (e.g. ion mobility unit) and then the second unit (e.g. second ion fragmentation), which is arranged after the first unit.

The term **"Mass Spectrometry"** ("Mass Spec" or "MS") or "mass spectrometric determination" relates to an analytical technology used to identify compounds by their mass. MS is a methods of filtering, detecting, and measuring ions based on their mass-to-charge ratio, or "m/z". MS technology generally includes (1) ionizing the compounds to form charged compounds; and (2) detecting the molecular weight of the charged compounds and calculating a mass-to-charge ratio. The compounds may be ionized and detected by any suitable means. A "mass spectrometer" generally includes an ionizer and an ion detector. In general, one or more molecules of interest are ionized, and the ions are subsequently introduced into a mass spectrographic instrument where, due to a combination of magnetic and electric fields, the ions follow a path in space that is dependent upon mass ("m") and charge ("z"). The term "ionization" or "ionizing" refers to the process of generating an analyte ion having a net electrical charge equal to one or more electron units. Negative ions are those having a net negative charge of one or more electron units, while positive ions are those having a net positive charge of one or more electron units. The MS method may be performed either in "negative ion mode", wherein negative ions are generated and detected, or in "positive ion mode" wherein positive ions are generated and detected.

**"Tandem mass spectrometry"** or "MS/MS" involves multiple steps of mass spectrometry selection, wherein fragmentation of the analyte occurrs in between the stages. In a tandem mass spectrometer, ions are formed in the ion source and separated by mass-to-charge ratio in the first stage of mass spectrometry (MS1). Ions of a particular mass-to-charge ratio (precursor ions or parent ion) are selected and fragment ions (or daughter ions) are created by collision-induced dissociation, ion-molecule reaction, or photodissociation. The resulting ions are then separated and detected in a second stage of mass spectrometry (MS2). Since a mass spectrometer separates and detects ions of slightly different masses, it easily distinguishes different isotopes of a given element. Mass spectrometry is thus, an important method for the accurate mass determination and characterization of analytes, including but not limited to low-molecular weight analytes, peptides, polypeptides or proteins. Its applications include the identification of proteins and their post-translational modifications, the elucidation of protein complexes, their subunits and functional interactions, as well as the global measurement of proteins in proteomics. De novo sequencing of peptides or proteins by mass spectrometry can typically be performed without prior knowledge of the amino acid sequence.

**"Multiple reaction mode"** or "MRM" is a detection mode for a MS instrument in which a precursor ion and one or more fragment ions are selectively detected.

The term **"in vitro method"** is used to indicate that the method is performed outside a living organism and preferably on body fluids, isolated tissues, organs or cells.

The term **"random-excess mode" or "random-excess"** as used herein is a broad term and is to be given its ordinary and customary meaning to a person of ordinary skill in the art and is not to be limited to a special or customized meaning. The term specifically may refer, without limitation, to a process that does not detect and/or measure at least one ion of the at least one analyte of interest sequentially, which can mean starting from the beginning and proceeding step by step. Random access or direct access may refer to the ability to access an arbitrary analyte of interest and/or sample of a sequence in equal time or any date from a population of addressable analyte of interest and/or sample roughly as easily and efficiently as any other, no matter how many analytes of interest and/or samples may be in the set.

A **"kit"** is any manufacture (e.g., a package or container) comprising at least one reagent, e.g., a medicament for treatment of a disorder, or a probe for specifically detecting a biomarker gene or protein of the invention. The kit is preferably promoted, distributed, or sold as a unit for performing the methods of the present invention. Typically, a kit may further comprise carrier means being compartmentalized to receive in close confinement one or more container means such as vials, tubes, and the like. In particular, each of the container means comprises one of the separate elements to be used in the method of the aspect. Kits may further comprise one or more other reagents including but not limited to reaction catalyst. Kits may further comprise one or more other containers comprising further materials including but not limited to buffers, diluents, filters, needles, syringes, and package inserts with instructions for use. A label may be present on the container to indicate that the composition is used for a specific application, and may also indicate directions for either in vivo or in vitro use. The computer program code may be provided on a data storage medium or device such as a optical storage medium (e.g., a Compact Disc) or directly on a computer or data processing device. Moreover, the kit may, comprise standard amounts for the analytes of interest for calibration purposes.

The system described herein may have the following features:
The analyte of interest may be artificial or a native.

The clinical diagnostic system may be free of a liquid chromatography (LC) unit and/or high pressure liquid chromatography (HPLC) unit and/or gas chromatography (GC) unit.

The biological sample may be prepared in a random-excess mode. The meaning of a random-excess mode is known for a skilled person and thus not explained in detail. The clinical diagnostic system is automated, preferably fully or partially automated.

The biological sample may be obtained from a patient sample, which is selected from a group consisting of serum, plasma, tissue, liquor, breath, sweat, sputum and whole blood sample from an individual.

The individual may be a mammal, preferably a human.

The at least one analyte of interest may be a small molecule.

The at least one analyte of interest may have a molar mass of smaller than m/z (mass-to-charge ratio) = 2000, preferably smaller than m/z =1000 or smaller than m/z =900 or smaller than m/z =800 or smaller than m/z =700 or smaller than m/z =600 or smaller than m/z =500 or smaller than m/z =400 or smaller than m/z =300 or smaller than m/z =200 or smaller than m/z =100 or smaller than m/z =90.

The at least one analyte of interest may not be a large molecule having a m/z ratio of < 2000, preferably the at least one analyte of interest may be selected from the following group: testosterone, vitamin D, cyclosporine A, lidocaine, sirolimus, abeta 42.

The at least one analyte of interest may be selected from the group consisting of nucleic acid, amino acid, peptide, protein, metabolite, hormones, fatty acid, lipid, carbohydrate, steroid, ketosteroid, secosteroid, a molecule characteristic of a certain modification of another molecule, a substance that has been internalized by the organism, a metabolite of such a substance and combination thereof.

The biological samples may comprise the at least one analyte of interest and a matrix, wherein preferably the matrix may comprise the following components or combinations thereof: inorganic salt, organic salt component, protein, solvents polymer, small molecule.

An inorganic salt may be e.g. (for example) sodium chloride.

An organic salt component may be e.g. HEPES ((4-(2-hydroxyethyl)-1-piperazineethanesulfonic acid) or citric acid.

A protein may be e.g. human serum albumin.

A solvent may be e.g. water, methanol and/or alcohol.

A polymer may be e.g. DNA and/or polyacrylamide.

A small molecule may be e.g. formaldehyde.

The matrix may separated from the at least one analyte of interest in the sample preparation station.

The clinical diagnostic system comprises a sample preparation station. The sample preparation station is for the automated preparation of biological samples. The at least one biological sample may comprise at least one analyte of interest.

The sample preparation station may comprise at least one of the following units or combinations thereof: sample delivery unit, sample pre-treatment unit, sample analytical unit.

The sample preparation station is automated, specifically partially or fully automated.

The sample preparation station may be configured for removing or at least reducing interfering matrix components in the biological sample and/or enriching analytes of interest in the biological sample. Additionally, the sample preparation station may comprise at least one of the following units or combinations thereof: sample delivery unit, sample pre-treatment unit, sample analytical unit

The sample delivery unit may comprise sample collection and/or preparation and/or transportation, e.g. including sample data preparation. This unit may consist of or comprise pipettors, gripper, conveyor and liquid handling robotors.

The sample pre-treatment unit comprises at least one unit or more than one units selected from the following group: magnetic bead handling unit, internal standard handling unit, supporting compound handling unit, pipetting unit, fluid transport unit, reaction container transporting mechanism unit, enrichment handling unit, solvent evaporation unit, derivatization unit.

The magnetic bead handling unit may be configured for treating samples with reagents comprising magnetic beads carrying analyte and/or matrix selective groups for extracting/enriching analytes of interest and removing or at least reducing matrix components. **In** particular, the magnetic bead handling unit may comprise at least one magnetic or electromagnetic workstation for holding at least one reaction container and for manipulating magnetic beads added to a sample or samples contained therein. The magnetic bead handling unit may further comprise a mixing mechanism for mixing fluids and/or resuspending the magnetic beads in the reaction container(s), e.g. by shacking or agitating the reaction container(s), e.g. by an eccentric rotation mechanism. Alternatively the bead handling unit may be a flow-through system where the magnetic beads are captured in a channel or capillary flow-through device. According to this embodiment, capturing, washing and releasing of analytes can be done by repeatedly magnetically capturing and releasing beads in a flow-through channel.

The magnetic bead handling unit may be configured for treating the biological sample with magnetic beads carrying analyte and/or matrix selective groups.

Magnetic or paramagnetic beads carrying analyte-selective groups may be added in the magnetic bead handling unit to the sample. The addition of the magnetic beads may comprise agitation or mixing. A pre-defined incubation period for capturing the analyte(s) of interest on the bead may follow. The workflow, which takes place in the magnetic bead handling unit, may comprise a washing step (W1) after incubation with the magnetic beads. Depending on the analyte(s) one or more additional washing steps (W2) may be performed. One washing step (W1, W2) may comprise a series of steps including magnetic bead separation by a magnetic bead handling unit comprising magnets or electromagnets, aspiration of liquid, addition of a washing buffer, resuspension of the magnetic beads, another magnetic bead separation step and another aspiration of the liquid. Moreover washing steps may differ in terms of type of solvent (water/organic/salt/pH), apart from volume and number or combination of washing cycles. It is well-known to the skilled person how to choose the respective parameters. The last washing step (W1, W2) may be followed by the addition of an elution reagent followed by resuspension of the magnetic beads and a pre-defined incubation period for releasing the analyte(s) of interest from the magnetic beads. The bound-free magnetic beads may then be separated and the supernatant containing derivatized analyte(s) of interest may be captured.

The magnetic beads carrying matrix-selective groups may be added in the magnetic bead handling unit to the sample. The addition of the magnetic beads may comprise agitation or mixing. A pre-defined incubation period for capturing the matrix on the bead may follow. Here, the analyte of interest may not bind to the magnetic beads but remains in the supernatant. Thereafter, the magnetic beads may be separated and the supernatant containing the enriched analyte(s) of interest may be collected.

The supernatant may be subjected to enrichment handling unit performing e.g. a second enrichment workflow. Here, the supernatant may be transferred to the at least one selectivity enhancer station or may be transferred to the at least one selectivity enhancer station after a dilution step by addition of a dilution liquid. Different elution procedures/reagents may also be used, by changing e.g. the type of solvents (water/organic/salt/pH/micoparticles) and volume. The various parameters may be well-known to the skilled person and easily chosen.

In alternative or in addition to magnetic bead handling unit, other techniques and/or units may be used such as protein precipitation followed by centrifugation, cartridge based solid phase extraction, pipette tip based solid phase extraction, liquid liquid extraction, affinity based extraction (immunosorption, molecular imprints, aptamers, etc).

The magnetic bead handling unit may be arranged after the sample delivery unit and before the sample analytical unit.

The internal standard handling unit may consist of a reservoir in which the internal standard with a defined concentration for the respective analyte is stored. From this reservoir a dedicated proportion may be taken out and given to the native/ or prepared sample (liquid to liquid or gasous to gasous).

The internal standard handling unit may be configured for the addition of at least one internal standard and/or standard addition.

The internal standard handling unit may be arranged after the sample delivery unit and before the sample analytical unit.

The supporting compound handling unit may be configured for the addition of supporting compounds to release the analyte from a respective binder. The binder may be Vitamin-D binding protein or albumin for Vitamin D or linoleic acid as analyte of interest.

Supporting compounds may be selected from the following group: methanol, NaOH, Ethylenecarbonat, trifluoroacetic acid (TFA), formic acid, bovine serum albumin (BSA), Ammoniumfluoride.

The supporting compound handling unit may be configured for the addition of supporting compounds or ion supporting molecules, e.g. in order to release the at least one analyte of interest from a binder, e.g. methanol or NaOH, or from a matrix or in order to add a matrix, e.g. 2,5-Dihydroxybenzoesäure (DHB) for MALDI applications or treat the analyte with a respective derivatization agent to change the chemical and/or physical properties of the analyte and/or the matrix.

The supporting compound handling unit may be arranged after the sample delivery unit and before the sample analytical unit.

The pipetting unit can consit of or may comprise a flow through needle or a direct dispensing unit to take liquid from one place to another with a defined volume. The pipetting unit can also consist of or may comprise a droplet ejecting unit driven by supersonic or piezo driven. The pipetting unit can pipett samples as well as supporting liquids.

The pipetting unit may be arranged after the sample delivery unit and before the sample analytical unit. The sample analytical unit can also consist of or comprises several independend reagent pipetting units which are independend of the mentioned pipetting unit.

The fluid transport unit may be configured for adding/removing fluids, such as samples, reagents, wash fluids, suspension fluids, into/from the reaction container(s) by delivering the reagents through tubings.

The fluid transport unit may be arranged after the sample delivery unit and before the sample analytical unit.

The one or more pipetting units and/or fluid transport units may be configured for adding, mixing or removing fluids.

In embodiments, the reaction container transporting mechanism unit. The transport can be achieved by convoyer or a gripping system which can handle reaction vessels.

The reaction container transporting mechanism unit may be configured for delivering sample or to the following process station, preferably the ion generation station.

The reaction container transporting mechanism unit may be arranged after the sample delivery unit and before the sample analytical unit.

The sample pre-treatment unit may comprise the enrichment handling unit. The enrichment can be performed by concentration of the sample through reducing the volume of solvent by e.g. evaporation or by attaching the analyte on a solid supported microparticles. Another enrichment possibility may be a precipitation of the analyte in its surrounding liquid by e.g. changing temperature or solvent exchange by following solid-liquid separation. The sample pre-treatment unit may comprise the derivatization unit which consists of or comprises a pipetting unit with heating and cooling capabilities. In this unit, a derivatization reagent or derivatization reagents can be used.

The derivatization reagents may be selected from the group consisting of: dansylchloride, carbamic acid, N-[2-[[[2-(diethylamino)ethyl]amino]carbonyl]-6-quinolinyl]-, 2,5-dioxo-1-pyrrolidinyl ester (RapiFluor-MS), 4-substituted 1,2,4-triazoline-3,5-diones (Cookson-type reagents), 4-Phenyl-1,2,4-triazolin-3,5-dion-derivative (Amplifex Diene), 1-propanaminium, 3-(aminooxy)-N,N,N-trimethyl-compound comprising an appropriate counter ion, e.g. bromide, chloride, iodine, etc. (Amplifex Keto), acethydrazide trimethylammonium chloride (Girard T), 1-(carboxymethyl)pyridinium chloride hydrazide (Girard P) and pyridiyl amine.

The enrichment handling unit may be arranged after the sample delivery unit and before the sample analytical unit.

The sample pre-treatment unit may comprise the solvent evaporation unit. The solvent evaporation unit can be part of the enrichment handling unit.

The solvent evaporation unit may be arranged after the sample delivery unit and before the sample analytical unit.

The addition of an hemolysis reagent can be performed in the sample pre-treatment unit.

The addition of enzymatic reagents to the sample, e.g. urine sample, can be performed in the sample pre-treatment unit.

The addition of at least one derivatisation reagent to the sample, e.g. urine sample, can be performed in the sample pre-treatment unit.

The analyte of interest may comprise a functional group. The functional group may be capable of reacting with a reactive unit Q of the derivatisation reagent.

The functional group may be selected from the group consisting of carbonyl group, diene group, hydroxyl group, amine group, imine group, ketone group, aldehyde group, thiol group, diol group, phenolic group, expoxid group, disulfide group, nucleobase group, carboxylic acid group, terminal cysteine group, terminal serine group and azide group.

The analyte molecule may comprise a carbonyl group as functional group which is selected from the group consisting of a carboxylic acid group, aldehyde group, keto group, a masked aldehyde, masked keto group, ester group, amide group, and anhydride group. Aldoses (aldehyde and keto) exist as acetal and hemiacetals, a sort of masked form of the parent aldehyde/ keto.

The carbonyl group may be an amide group, the skilled person is well aware that the amide group as such is a stable group, but that it can be hydrolyzed to convert the amide group into an carboxylic acid group and an amino group. Hydrolysis of the amide group may be achieved via acid/base catalysed reaction or by enzymatic process either of which is well-known to the skilled person. The carbonyl group is a masked aldehyde group or a masked keto group, the respective group is either a hemiacetal group or acetal group, in particular a cyclic hemiacetal group or acetal group.

The acetal group may be converted into an aldehyde or keto group before reaction with the compound.

The carbonyl group may be a keto group. The keto group may be transferred into an intermediate imine group before reacting with the reactive unit of compounds. The analyte molecule may comprise one or more keto groups which is a ketosteroid. The ketosteroid may be selected from the group consisting of testosterone, epitestosterone, dihydrotestosterone (DHT), desoxymethyltestosterone (DMT), tetrahydrogestrinone (THG), aldosterone, estrone, 4-hydroxyestrone, 2-methoxyestrone, 2-hydroxyestrone, 16-ketoestradiol, 16-alpha-hydroxyestrone, 2-hydroxyestrone-3-methylether, prednisone, prednisolone, pregnenolone, progesterone, dehydroepiandrosterone (DHEA), 17-hydroxypregnenolone, 17-hydroxyprogesterone, androsterone, epiandrosterone, Δ4-androstenedione, 11-deoxycortisol, corticosterone, 21-deoxycortisol, 11-deoxycorticosterone, allopregnanolone and aldosterone.

The carbonyl group may be a carboxyl group. The carboxyl group may react directly with the compound or it is converted into an activated ester group before reaction with the compound. The analyte molecule may comprise one or more carboxyl groups which is selected from the group consisting of Δ8-tetrahydrocannabinolic acid, benzoylecgonin, salicylic acid, 2-hydroxybenzoic acid, gabapentin, pregabalin, valproic acid, vancomycin, methotrexate, mycophenolic acid, montelukast, repaglinide, furosemide, telmisartan, gemfibrozil, diclofenac, ibuprofen, indomethacin, zomepirac, isoxepac and penicillin. The analyte molecule may comprise one or more carboxyl groups is an amino acid selected from the group consisting of arginine, lysine, aspartic acid, glutamic acid, glutamine, asparagine, histidine, serine, threonine, tyrosine, cysteine, tryptophan, alanine, isoleucine, leucine, methionine, phenyalanine, valine, proline and glycine.

The carbonyl group may be an aldehyde group. The aldehyde group may be transferred into an intermediate imine group before reacting with the reactive unit of compounds. The analyte molecule comprising one or more aldehyde groups may be selected from the group consisting of pyridoxal, N-acetyl-D-glucosamine, alcaftadine, streptomycin and josamycin.

The carbonyl group may be a carbonyl ester group. The analyte molecule may comprise one or more ester groups which is selected from the group consisting of cocaine, heroin, Ritalin, aceclofenac, acetylcholine, amcinonide, amiloxate, amylocaine, anileridine, aranidipine artesunate and pethidine.

The carbonyl group may be an anhydride group. The analyte molecule may comprise one or more anhydride groups which is selected from the group consisting of cantharidin, succinic anhydride, trimellitic anhydride and maleic anhydride.

The analyte molecule may comprise one or more diene groups, in particular to conjugated diene groups, as functional group. The analyte molecule may comprise one or more diene groups which is a secosteroid. The secosteroid may be selected from the group consisting of cholecalciferol (vitamin D3), ergocalciferol (vitamin D2), calcifediol, calcitriol, tachysterol, lumisterol and tacalcitol. In particular, the secosteroid may be vitamin D, in particular vitamin D2 or D3 or derivates thereof. In particular embodiments, the secosteroid may be selected from the group consisting of vitamin D2, vitamin D3, 25-hydroxyvitamin D2, 25-hydroxyvitamin D3 (calcifediol), 3-epi-25-hydroxyvitamin D2, 3-epi-25-hydroxyvitamin D3, 1,25-dihydroxyvitamin D2, 1,25-dihydroxyvitamin D3 (calcitriol), 24,25-dihydroxyvitamin D2, 24,25-dihydroxyvitamin D3. The analyte molecule may comprise one or more diene groups which is selected from the group consisting of vitamin A, tretinoin, isotretinoin, alitretinoin, natamycin, sirolimus, amphotericin B, nystatin, everolimus, temsirolimus and fidaxomicin.

The analyte molecule may comprise one or more hydroxyl group as functional group. The analyte molecule may comprise a single hydroxyl group or two hydroxyl groups. In embodiments wherein more than one hydroxyl group is present, the two hydroxyl groups may be positioned adjacent to each other (1,2-diol) or may be separated by 1, 2 or 3 C atoms (1,3-diol, 1,4-diol, 1,5-diol, respectively). The analyte molecule comprises a 1,2-diol group. In embodiments, wherein only one hydroxyl group is present, said analyte may be selected from the group consisting of primary alcohol, secondary alcohol and tertiary alcohol. In embodiments, wherein the analyte molecule comprises one or more hydroxyl groups, the analyte may be selected from the group consisting of benzyl alcohol, menthol, L-carnitine, pyridoxine, metronidazole, isosorbide mononitrate, guaifenesin, clavulanic acid, Miglitol, zalcitabine, isoprenaline, aciclovir, methocarbamol, tramadol, venlafaxine, atropine, clofedanol, alpha-hydroxyalprazolam, alpha-Hydroxytriazolam, lorazepam, oxazepam, Temazepam, ethyl glucuronide, ethylmorphine, morphine, morphine-3-glucuronide, buprenorphine, codeine, dihydrocodeine, p-hydroxypropoxyphene, O-desmethyltramadol, Desmetramadol, dihydroquinidine and quinidine. In embodiments of the first aspect of the present invention, wherein the analyte molecule comprises more than one hydroxyl groups, the analyte is selected from the group consisting of vitamin C, glucosamine, mannitol, tetrahydrobiopterin, cytarabine, azacitidine, ribavirin, floxuridine, Gemcitabine, Streptozotocin, adenosine, Vidarabine, cladribine, estriol, trifluridine, clofarabine, nadolol, zanamivir, lactulose, adenosine monophosphate, idoxuridine, regadenoson, lincomycin, clindamycin, Canagliflozin, tobramycin, netilmicin, kanamycin, ticagrelor, epirubicin, doxorubicin, arbekacin, streptomycin, ouabain, amikacin, neomycin, framycetin, paromomycin, erythromycin, clarithromycin, azithromycin, vindesine, digitoxin, digoxin, metrizamide, acetyl digitoxin, deslanoside, Fludarabine, clofarabine, gemcitabine, cytarabine, capecitabine, vidarabine, and plicamycin.

The analyte molecule may comprise one or more thiol group (including but not limited to alkyl thiol and aryl thiol groups) as functional group. The analyte molecule may comprise one or more thiol groups is selected from the group consisting of thiomandelic acid, DL-captopril, DL-thiorphan, N-acetylcysteine, D-penicillamine, glutathione, L-cysteine, zofenoprilat, tiopronin, dimercaprol, succimer.

The analyte molecule may comprise one or more disulfide group as functional group. The analyte molecule may comprise one or more disulfide groups which is selected from the group consisting of glutathione disulfide, dipyrithione, selenium sulfide, disulfiram, lipoic acid, L-cystine, fursultiamine, octreotide, desmopressin, vapreotide, terlipressin, linaclotide and peginesatide. Selenium sulfide can be selenium disulfide, SeS₂, or selenium hexasulfide, Se₂S₆.

The analyte molecule may comprise one or more epoxide group as functional group. The analyte molecule may comprise one or more epoxide groups which is selected from the group consisting of Carbamazepine-10,11-epoxide, carfilzomib, furosemide epoxide, fosfomycin, sevelamer hydrochloride, cerulenin, scopolamine, tiotropium, tiotropium bromide, methylscopolamine bromide, eplerenone, mupirocin, natamycin, and troleandomycin.

The analyte molecule may comprise one or more phenol groups as functional group. Analyte molecules may comprise one or more phenol groups are steroids or steroid-like compounds. The analyte molecule may comprise one or more phenol groups which is a steroid or a steroid-like compound having an A-ring which is sp² hybridized and an OH group at the 3 -position of the A-ring. The steroid or steroid-like analyte molecule may be selected from the group consisting of estrogen, estrogen-like compounds, estrone (El), estradiol (E2), 17a-estradiol, 17b-estradiol, estriol (E3), 16-epiestriol, 17-epiestriol, and 16, 17-epiestriol and/or metabolites thereof. In embodiments, the metabolites are selected from the group consisiting of estriol, 16-epiestriol (16-epiE3), 17-epiestriol (17-epiE3), 16,17-epiestriol (16,17-epiE3), 16-ketoestradiol (16-ketoE2), 16a-hydroxyestrone (16a-OHEl), 2-methoxyestrone (2-MeOEl), 4-methoxyestrone (4-MeOEl), 2-hydroxyestrone-3-methyl ether (3-MeOEl), 2-methoxyestradiol (2-MeOE2), 4-methoxyestradiol (4-MeOE2), 2-hydroxyestrone (2-OHE1), 4-hydroxyestrone (4-OHE1), 2-hydroxyestradiol (2-OHE2), estrone (El), estrone sulfate (Els), 17a- estradiol (E2a), 17b-estradiol (E2B), estradiol sulfate (E2S), equilin (EQ), 17a-dihydroequilin (EQa), 17b-dihydroequilin (EQb), Equilenin (EN), 17-dihydroequilenin (ENa), 17α-dihydroequilenin, 17β-dihydroequilenin (ENb) , Δ8,9-dehydroestrone (dEl), Δ8,9-dehydroestrone sulfate (dEls), Δ9-tetrahydrocannabinol, mycophenolic acid. β or b can be used interchangeable. α and a can be used interchangeable.

The analyte molecule may comprise an amine group as functional group. The amine group may be an alkyl amine or an aryl amine group. The analyte may comprise one or more amine groups which is selected from the group consisting of proteins and peptides. The analyte molecule may comprise an amine group which is selected from the group consisting of 3,4-methylenedioxyamphetamine, 3,4-methylenedioxy-N-ethylamphetamine, 3,4-methylenedioxymethamphetamine, Amphetamine, Methamphetamine, N-methyl-1,3-benzodioxolylbutanamine, 7-aminoclonazepam, 7-aminoflunitrazepam, 3,4-dimethylmethcathinone, 3-fluoromethcathinone, 4-methoxymethcathinone, 4-methylethcathinone, 4-methylmethcathinone, amfepramone, butylone, ethcathinone, elephedrone, methcathinone, methylone, methylenedioxypyrovalerone, benzoylecgonine, dehydronorketamine, ketamine, norketamine, methadone, normethadone, 6-acetylmorphine, diacetylmorphine, morphine, norhydrocodone, oxycodone, oxymorphone, phencyclidine, norpropoxyphene, amitriptyline, clomipramine, dothiepin, doxepin, imipramine, nortriptyline, trimipramine, fentanyl, glycylxylidide, lidocaine, monoethylglycylxylidide, N-acetylprocainamide, procainamide, pregabalin, 2-Methylamino-1-(3,4-methylendioxyphenyl)butan, N-methyl-1,3-benzodioxolylbutanamine, 2-Amino-1-(3,4-methylendioxyphenyl)butan, 1,3-benzodioxolylbutanamine, normeperidine, O-Destramadol, desmetramadol, tramadol, lamotrigine, Theophylline, amikacin, gentamicin, tobramycin, vancomycin, Methotrexate, Gabapentin sisomicin and 5-methylcytosine.

The analyte molecule may be a carbohydrate or substance having a carbohydrate moiety, e.g. a glycoprotein or a nucleoside. The analyte molecule may be a monosaccharide, in particular selected from the group consisting of ribose, desoxyribose, arabinose, ribulose, glucose, mannose, galactose, fucose, fructose, N-acetylglucosamine, N-acetylgalactosamine, neuraminic acid, N-acetylneurominic acid, etc.. In embodiments, the analyte molecule is an oligosaccharide, in particular selected from the group consisting of a disaccharide, trisaccharid, tetrasaccharide, polysaccharide. In embodiments of the first aspect of the present invention, the disaccharide is selected from the group consisting of sucrose, maltose and lactose. The analyte molecule may be a substance comprising above described mono-, di-, tri-, tetra-, oligo- or polysaccharide moiety.

The analyte molecule may comprise an azide group as functional group which is selected from the group consisting of alkyl or aryl azide. The analyte molecule may comprise one or more azide groups which is selected from the group consisting of zidovudine and azidocillin.

Such analyte molecules may be present in biological or clinical samples such as body liquids, e.g. blood, serum, plasma, urine, saliva, spinal fluid, etc., tissue or cell extracts, etc. The analyte molecule(s) may be present in a biological or clinical sample selected from the group consisting of blood, serum, plasma, urine, saliva, spinal fluid, and a dried blood spot. The analyte molecules may be present in a sample which is a purified or partially purified sample, e.g. a purified or partially purified protein mixture or extract.

The analyte of interest may be a steroid.

The steroid may be selected from the group consisting of Cortisol, DHEA-S, Estradiol, Progesterone, Testosterone, 17-Hydroxyprogesterone, Aldosterone, Androstendione, DHEA, Dihydrotestosterone and Cortisone.

The analyte of interest may be a Vitamin D.

The Vitamin D may be selected from the group consisting of 25(OH)D2, 25(OH)D3, 24,25(OH)2D2, 24,25(OH)2D3, 1,25(OH)2D2 and 1,25(OH)2D3. A skilled person knows the abbreviations mentioned above for Vitamin D.

The analyte of interest may be selected from the group consisting of Cyclosporine A, Everolimus, Sirolimus, Tacrolimus, Acetaminophen, Salicylate, Theophylline and Digoxin.

The analyte of interest may be selected from the group consisting of Phenytoin, Valproic acid, Phenytoin, Levetiracetam, Carbamazepine, Carbamazepine-10,11-epoxide, Phenobarbital, Primidone, Gabapentin, Zonisamid, Lamotrigine and Topiramateand.

The analyte of interest may be selected from the group consisting of Gentamicin, Tobramycin, Amikacin, Vancomycin, Piperacilline (Tazobactam), Meropenem and Linezolid.

The analyte of interest may be selected from the group consisting of Methotrexate, Voriconazole, Mycophenolic acid and Mycophenolic acid-glucuronide.

The analyte of interest may be selected from the group consisting of Buprenorphine, 6-monoacatylmorphine, Codeine, Dihydrocodeine, Morphine, Morphine-3-glucuronide and Tramadol.

The analyte of interest may be selected from the group consisting of Acetylfentanyl, Carfentanil, Fentanyl, Hydrocodone, Norfentanyl, Oxycodone and Oxymorphone.

The sample preparation station may comprise a sample analytical unit.

The sample analytical unit may comprise at least one unit or more than one units or a combination of the units selected from the group: a solid-liquid support analyte enrichment unit, solid-liquid support matrix depletion unit, gaseous sample enrichment or matrix separation unit, solid sample on solid support unit.

The solid-liquid support analyte enrichment unit may be e.g. SPE (solid phase extraction), SLE (Solid liquid extraction), SPME (Solid Phase Micro Extraction). This unit may bring the sample in contact to a supporting liquid or uses the liquid in which the sample is already in. Furthermore a solid may get into contact with the sample and due to surface adsorption and or absorption a separation of compunds from the sample is achieved. The solid can be removed and the supernatant or the solid particles can be processed further by e.g. washing and/or extraction. The unit may be capable to handle this type of extraction in an automated way from sample in, extraction and or washing followed by transferring the processed sample further.

The solid-liquid support analyte enrichment unit may be selected from the group consisting of solid-phase extraction (SPE), micro particle, liquid-liquid extraction (LLE), liquid chromatography (LC), high pressure liquid chromatography (HPLC) and combinations thereof.

The solid-liquid support matrix depletion unit may capable in a similar manner to the solid-liquid support analyte enrichment unit to remove unwanted matrix components from the sample by e.g. microparticles or a prepacked column. The unwanted matrix may be by this unit bound to the particles or gets washed within the supernatenat of the particles.

The solid-liquid support matrix depletion unit may be selected form the group consisting of solid-phase extraction (SPE), micro particle and liquid-liquid extraction (LLE).

The gaseous sample enrichment or matrix separation unit may consist of or may comprise a SLE unit or a heating/cooling unit in which analytes and/or matrix compounds within a analytical sample gets separated. Further, more filtering devices or units to separate aerosols e.g. water droplets ranging from 10 nm-10 mm may get filtered off.

The solid sample on solid support unit may consist of or may comprise a pipetting unit if liquid samples are handled or a robotic arm for solid samples. The solid support can consist of or may comprise a metal plate or a solid strip. The sample can be dried if a liquid was placed and/or mixed on the solid support with other reagents. An example is the MALDI (matrix assisted laser desorption) in which a pre mixed analyte and matrix compounds e.g. DHB are pipetted to get onto a steel plate and evaporate till dryness follows by laser irridation and mass spectrometrical analysis.

The solid sample on solid support unit may be imaging of cross sections and mineral analysis.

The gaseous sample enrichment or matrix separation unit may be inert gas stripping or headspace extraction or gas adsorption supported by liquid and/or solid e.g. activated charcoal or combinations thereof.

The clinical diagnostic system may comprise a ion generation station. The ion generation station may be configured for generating at least one ion or ions of the at least one analyte of interest.

The ion or ions of the at least one analyte of interest may be in the gaseous state, preferably after leaving the ion generation station.

The ion generation station may be arranged between the sample preparation station and the at least one selectivity enhancer station. This can mean that the ion generation station is coupled with the sample preparation station and is coupled to the at least one selectivity enhancer station.

The ion generation station may comprise a steady analyte ion supply unit. The laser desorption ionzation (LDI) may be a system which generates ion/ ionization process driven by the use of the laser energy. The energy may be taken up by the molecule and/or a supporting material to separate charges and gererate ions.

The dielectric barrier liquid flow may be characterized by the presence of one or more insulating layers (dielectric barriers) in the current path between metal electrodes in addition to the discharge space. The dielectric barriers usually use several common insulation materials, for example glass or silica glass, ceramic materials, mica, and thin enamel or polymer layers. There are two basic configurations for a DBD: planar and annular.

The surface plasma ionization may be made of or may comprise a source which provides a cold plasma by e.g. electrical discharge which is pointed towards a surface bound analyte. The analyte may get ionized by the cold plasma inherent ions.

The steady analyte ion supply unit may be selected from the group consisting of flow injection (preferably flow injection using ESI or APCI or Nano Spray), paper spray, electron ionization (EI), matrix assisted ionization (MAI), chemical ionization (CI), e.g. proton-transfer-reaction (PTR), radioactive supported ionization, e.g. using ⁶³Ni and combinations thereof.

The flow injection may relate to a direct sample to ESI/ APCI spray needle contact. The sample may get without any further processing through the ion source.

The flow injection may be an electrospray ionization (ESI).

The ESI may be nanoESI.

The nanoESI may be static nanoESI.

The flow injection may be atmospheric pressure chemical ionization (APCI).

The the clinical diagnostic system may comprise high-field asymmetric-waveform ion-mobility spectrometry (FAIMS).

The paper spray may relate to a paper strip which is shaped on one side with a tip or cone. The analyte may be applied on the tip surface and wetted with solvent. On the opposite side of the paper a high voltage may be applied (around 0,5-5kV) and the tip may be placed in front of the mass spectrometer.

The electron ionization (EI) may use free electrons from a glowing wire which are accelerated by an electromagnetic field. The electron beam may hit the target analyte and may ionize it therefore by forming radical cations and or anions.

The matrix assisted ionization (MAI) may uses a solide matrix e.g. 3-NBN which dissociates by thermal energy and or electric fields and therefore ionized the molecule of interest which is in close contact to the matrix (e.g. by co-precipitation).

The chemical ionization (CI) may use an electron beam to ionize a supporting gas (e.g. methane or ammonia) which further ionizes the target analyte.

The radioactive supported ionization may use the alpha radiation of e.g. ⁶³Ni as source for ions which therefore may ionize a target molecule within the radion beam of the radioactive material.

The clinical diagnostic system may comprise a at least one selectivity enhancer station. The clinical diagnostic system can comprise more than one selectivity enhancer station, e.g. 2 or 3 or 4 or 5 or 6, for enhancing the selectivity of the at least one analyte of interest. In case of more than 2 selectivity enhancer station, the selectivity enhancer stations may be coupled to each other one after the other. The at least one selectivity enhancer station separates ions may be based on the respective ion size. The at least one selectivity enhancer station may comprise an ion mobility unit for separation ions with respect to their ion mobility using electric and/or radio frequency fields (AC/DC and/or RF) applied to one or more electrodes. The clinical diagnostic system may comprise exactly one selectivity enhancer station.

The at least one selectivity enhancer station may comprise at least one ion storage unit and/or the at least one ion transportation unit, and an ion selection unit which separates ions based on their m/z (mass over charge ratio). Preferably, the ion storage unit and/or the at least one ion transportation unit may have no ion separation capability.

The at least one selectivity enhancer station may comprise exactly one ion storage unit and/or exactly one ion transportation unit.

The ions with similar or equal ion mobility unit may bunch together in the ion mobility unit.

The ion mobility unit may perform a gas phase separation of a mixture of ions.

The ions may have a mass to charge ratio the range of 1 to about 100,000 in the ion mobility unit.

The ions may have a drift time through the ion mobility unit of about 0 to about 60 seconds.

The at least a portion of the ion mobility unit may be maintained at a pressure in the range of about 10-3 torr to atmospheric pressure.

The ions in the ion mobility unit may be formed by using at least one of the following: photoionization, Corona discharge, laser ionization, electron impact, field ionization, chemical ionization, and electrospray.

The ions may be formed outside of the ion mobility unit, preferably in the ion generation station.

The ion mobility unit may comprise a non-constant electric field applied to the ion mobility unit to form a potential gradient along a portion of the ion mobility unit so that ions with similar mobilities bunch together into sharper peaks while maintaining separation between other ions.

The potential gradient may be a DC gradient.

The potential gradient progressively may increase or decreases along the length of the device.

The potential gradient along a length of the device may be between 0 to about 5,000 volts/mm.

The ion mobility unit may use an electric field and/or a radio frequency fields (AC/DC and/or RF) applied to one or more electrodes.

The ion mobility unit may comprise a
- first pair of opposing electrode arrangements coupled to a voltage source and that confines ions between the first pair opposing electrode arrangements in a confinement volume portion of a confinement volume inwardly laterally in a first confinement direction with respect to a longitudinal ion propagation direction, each opposing electrode arrangement of the first pair including an arrangement of RF electrodes that receives an unbiased RF voltage from the voltage source having an alternate phase between adjacent RF electrodes of the arrangement of RF electrodes of the opposing electrode arrangement of the first pair that provides the confining of ions between the first pair of opposing electrode arrangements; and
- a second pair of opposing electrode arrangements coupled to the voltage source and separate from the first pair of opposing electrode arrangements and that confines the ions between the second pair of opposing electrode arrangements in the confinement volume inwardly laterally in a second confnement direction that complements the first confinement direction, each opposing electrode arrangement of the second pair including an arrangement of RF electrodes that receives an unbiased RF voltage from the voltage source having an alternate phase between adjacent RF electrodes of the arrangement of RF electrodes of the opposing electrode arrangement of the second pair; wherein the confnement volume defines a first ion conduit and the apparatus further comprises a second ion conduit separate and laterally spaced apart from the first ion conduit and wherein the second ion conduit includes a plurality of electrode arrangements and at least one the electrode arrangements of the second ion conduit is an opposing electrode arrangement of the first or second pairs of opposing electrode arrangements of the first ion conduit.

The ion mobility unit may comprise:
at least one surface;
a first plurality of continuous electrodes coupled to the at least one surface and in electrical communication with a radiofrequency (RF) voltage source, wherein an RF voltage applied to adjacent electrodes of the first plurality of electrodes by the RF voltage source is phase shifted on the adjacent electrodes of the first plurality of electrodes by approximately 180°; and
a second plurality of segmented electrodes coupled to the at least one surface and arranged in longitudinal sets between or adjacent to the first plurality of electrodes, the second plurality of segmented electrodes being further in electrical communication with an alternating current (AC) voltage source, wherein an AC voltage waveform applied to adjacent electrodes within a longitudinal set of the second plurality of segmented electrodes by the AC voltage source is phase shifted on the adjacent electrodes of the second plurality of electrodes by l°-359°.

The ion mobility unit may comprise a plurality of guard electrodes positioned on outer ends of the first and second plurality of electrodes on the at least one surface, the plurality of guard electrodes being further in electrical communication with a DC voltage source, wherein the plurality of guard electrodes generate electric fields that constrain ion motion towards the guard electrodes when receiving a constant DC voltage from the DC voltage source.

The AC voltage waveform may be a sine wave.

The AC voltage waveform may be the sum of more than one AC voltage waveform.

The AC voltage waveform applied to adjacent electrodes within a longitudinal set of the second plurality of segmented electrodes may be phase shifted on the adjacent electrodes of the second plurality of segmented electrodes in a repeating pattern.

The AC voltage waveform applied to adjacent electrodes within a longitudinal set of the second plurality of segmented electrodes may be phase shifted by approximately 45°, 90° or 120° on the adjacent electrodes of the second plurality of electrodes in a stepwise fashion.

The at least one surface may comprise a single and non-planar surface.

The single, non-planar surface may be one of the following shapes: curved, cylindrical, a spiral, a funnel, hemispherical, or elliptical.

The at least one surface may comprise two surfaces spaced apart from one another.

The two surfaces may be approximately parallel to one another.

A frequency of the applied AC voltage waveform may be selected from the range of 10 Hz - 200 kHz, and a frequency of the applied RF voltage may be selected from the range of 100 kHz - 5 MHz.

A frequency applied AC voltage waveform may be selected from the range of 1 Hz to 1 kHz.

A pressure range of the ion mobility unit may be from atmospheric pressure to 1 mtorr vacuum.

The ion mobility unit may comprise
at least one surface;
a plurality of segmented electrodes coupled to the at least one surface and arranged in one or more longitudinal sets, the plurality of segmented electrodes being in electrical communication with an alternating current (AC) voltage source and a radiofrequency (RF) voltage source;
wherein an AC voltage waveform applied to adjacent electrodes within a longitudinal set of the plurality of electrodes by the AC voltage source is phase shifted by l°-359°; and
wherein an RF voltage applied to adjacent electrodes of the plurality of electrodes by the RF voltage source is phase shifted by approximately 180°.The ion mobility unit may comprise a supporting gas, preferably with variable direction to the AC/DC and RF driven ion direction can be applied with angle towards the ion flow of 1° to 359°. Preferably, the angle towards the ion flow is 90° or 180°.

The supporting gas may be inert.

The supporting gas may be a non reactive gas, e.g. nitrogen, argon, xenon, krypton, SF6 and/or helium.

The supporting gas may be non reactive with pressure of 0-1 bar, preferably, 1 mbar - 500 mbar. By flowing the supporting gas the ions can be further separated with this alternative force toward the AC/DC RF driven ion movement.

The ion mobility unit may comprise
a. pair of surfaces;
b. arrays of electrodes coupled to the surfaces to which RF potentials are applied to at least one of the surfaces in order to create a pseudopotential that inhibits charged particles from approaching either of the surfaces; and
c. simultaneous application of DC potentials to control and restrict movement of ions in between the surfaces.
the arrays of electrodes comprise an array of inner electrodes, a first outer electrode array, and a second outer electrode array, wherein the inner and the outer array of electrodes extend substantially along the length of each surface.

The arrays of electrodes may comprise an array of inner electrodes, a first outer electrode array, and a second outer electrode array, wherein the inner and the outer array of electrodes extend substantially along the length of each surface.

The first outer electrode array may be positioned on one side of the array of inner electrodes, and the second outer electrode array may be positioned on the other side of the array of inner electrodes.

The DC potentials may be applied to the first and second outer electrode arrays and wherein the RF potentials, with a superimposed electric field, may be applied to the array of inner electrodes.

The RF potentials may be applied along with the DC potentials on the first and second outer electrode arrays.

The RF potentials may be applied to only one of the two surfaces.

The RF potentials may be applied to both of the two surfaces.

The RF on at least one inner electrode may be out of phase with its neighboring electrode.

Each inner electrode may be phase shifted with its neighboring inner electrode to form the pseudopotential.

The inner electrode may be approximately 180 degrees out of phase with its neighboring inner electrode to form the pseudopotential.

The ion mobility unit may further comprise multiple pairs of surfaces, wherein transfer of the ions may be allowed through an aperture and guided by a series of electrodes to move between different pairs of surfaces of the multiple pairs of surfaces.

The series of electrodes may have RF potential of alternating polarity to prevent loss of ions as the ions move between the different pairs of parallel surfaces.

The electrodes on the pair of surfaces may form at least one of the following configurations:
a. a substantially T-shaped configuration, allowing ions to be switched at a junction of the T-shaped configuration;
b. a substantially Y-shaped configuration, allowing ions to be switched at a junction of the Y-shaped configuration;
c. a substantially X-shaped or cross-shaped configuration, allowing ions to be switched at a junction of one or more sides of the X-shaped or cross-shaped configuration; and d. a substantially multidirectional shape, such as an asterisk (*) -shaped configuration, with multiple junction points, allowing ions to be switched at a junction to one or more sides of the configuration.

The electric field may allow the ions to move in a circular- shaped path, a rectangular-shaped path, or other irregular path, to allow the ions to make more than one transit.

The space between the surfaces may include an inert gas or a gas that ions react with.

The ion mobility unit may further comprise multiple cyclotron stages.

The multiple cyclotron stages may be arranged in a stack.

The ion mobility unit may be coupled to at least one of the following: a charge detector, an optical detector, and a mass spectrometer.

The ions may be introduced from outside the ion mobility unit and may be formed using at least one of the following: photoionization, Corona discharge, laser ionization, electron impact, field ionization, chemical ionization, and electrospray.

The electric field may be a static electric field or a dynamic electric field.

The static field may be a DC gradient and the dynamic field is a traveling wave.

The RF potentials, may have two or more distinct frequencies and different electric field strengths, are co-applied to the arrays of electrodes on the surfaces and with a pattern of application that creates a pseudopotential that inhibits charged particles from approaching one or both of the surfaces over a substantially greater m/z range than would be feasible with other RF potentials of a single frequency.

The surfaces may be one of the following: planar, parallel, and not flat. The selectivity enhancer station may comprise the ion mobility unit and at least one unit selected from the following group: ion transportation unit, ion fragmentation unit.

The ion mobility unit may be for spatial mobility filtering and/or temporal mobility filtering.

The ion transportation unit may be selected from the group consisting of ion funnels, step wave, S-lens, ion mirror, ion fight tube by differential potential, ion trap, multipole, e.g. quadrupole, non-contact ion transport system, e.g. direct source coupling in which a capillary (e.g. heated capillary) or two lenses with a electrostatic gradient may be a connection for two ion handling units (e.g. source and a quadrupole) and magnetic sector transport.

The ion fragmentation unit may be selected from the group consisting of collision-induced dissociation (CID), electron-capture dissociation (ECD), electron-transfer dissociation (ETD), photon dissociation and electron impact (EI).

The ion mobility unit may be arranged between the ion transportation unit and an ion fragmentation unit.

At least one selectivity enhancer station may comprise or may consist of one ion fragmentation unit or two ion fragmentation units or three ion fragmentation units or four ion fragmentation units. The ion mobility unit can be arranged between two ion fragmentation units, preferably the ion mobility unit can directly couplet to a first ion fragmentation unit and directly coupled to a second ion fragmentation unit.

The analyte of interest may pass through a first ion fragmentation unit, then the ion mobility unit, and then a second ion fragmentation unit.

The analyte of interest may pass through the ion transportation unit, then the ion mobility unit, and then a second ion fragmentation unit.

The first ion fragmentation unit may be selected from the group consisting of collision-induced dissociation (CID), electron-capture dissociation (ECD), electron-transfer dissociation (ETD), and photon dissociation, electron impact (EI).

The second ion fragmentation unit may be selected from the group consisting of ion trap, orbitrap, time-of-flight (TOF), magnetic sector, e.g. double focusing sector field (DFS).

The first ion fragmentation unit may be arranged before the ion mobility unit.

The second ion fragmentation unit may be arranged after the ion mobility unit.

The ion transportation unit may be arranged before the ion mobility unit, preferably before the first ion fragmentation unit.

The collision-induced dissociation (CID) may be related to the collision of the target analyte with a neutral gas (e.g. argon, nitrogen). The target analyte gets the energy for the collion due to a electric field driven velocity.

The electron-capture dissociation (ECD) may be related to a dissociation driven by electrons from a supporting component which is a odd-electron species.

The electron-transfer dissociation (ETD) may be related to an electron to be transferred to the positive precursor molecules radical anions are generated and put into the ion trap with them. During the ion/ion reaction an electron may be transferred to the positively-charged analyte molecule, causing fragmentation.

The photon dissociation may be related to the process of storing analyte ions (e.g. in a ion trap) followd by irridation of light with an energy to be capable to break the analyte bonds.

The ion trap may be related to a device which stores ions based upon their m/z ratio by modulating AC/DC and RF values which may be applied to the ion trap cell in which the ions are stored.

The time-of-flight (TOF) may be related to a time dependend technique in which the ions may be accellarated by electric fields with a defined time point for the start. The ions may reach the detector after certain time which is directly dependend on their m/z value.

The double focusing magnetic sector may be related to a combination of an permanent magnet and an electromagnetic magnet which directs the ions through a space and by the two sectors different m/z values can be separated.

The clinical diagnostic system may comprises an ion detection station. The ion detection station may be for detecting of at least one ion of the at least one analyte of interest.

The ion detection station may comprise at least one unit or more than one units selected from the group: faraday cup, microchannel plate detector (MCP), photomultiplier, orbitrap, electron multiplier, photographic plate.

The faraday cup may be related to a metal (conductive) cup designed to catch charged particles in vacuum. The resulting current can be measured and used to determine the number of ions or electrons hitting the cup.

The microchannel plate detector (MCP) may be related to a planar component used for detection of single particles (electrons, ions and neutrons).

The photomultiplier may be related to a device for which an analyte molecule hits a first dynode an therefore amplifies the hit process on the first dynode by ejection of multiple other charge units which therefore hits the next dynode etc. In the end a ion sensitive detector can record the multiplied signal.

The orbitrap may be related to an ion trap mass analyzer consisting of an outer barrel-like electrode and a coaxial inner spindle-like electrode that traps ions in an orbital motion around the spindle. The image current from the trapped ions may be detected and converted to a mass spectrum using the Fourier transform of the frequency signal.

The electron multiplier may be related to a vacuum-tube structure that multiplies incident charges. In a process called secondary emission, a single electron can, when bombarded on secondary-emissive material, induce emission of roughly 1 to 3 electrons. If an electric potential is applied between this metal plate and yet another, the emitted electrons will accelerate to the next metal plate and induce secondary emission of still more electrons. This can be repeated a number of times, resulting in a large shower of electrons all collected by a metal anode, all having been triggered by just one.

The photographic plate may be a ion sensitive plated by a chemical reaction. This plate can fix the ion image after followed chemical reactions (e.g. like a photographic process with silver ions).

The ion detection station may be a mass spectrometer. The ion detection station can perform mass spectrometry.

The ion detection station may be a tandem mass spectrometer.

Most common sample workflows in MS include sample preparation and/or enrichment steps, wherein the analyte(s) of interest are separated from the matrix using gas or liquid chromatography. The inventor surprisingly found that a clinical diagnostic system of the first aspect of the invention, which is preferably free of an chromatography unit, e.g. as part of the selectivity enhancer station, shows advantages compared to a system comprising a chromatography unit. This is mostly performed by removing any parts in which mechanical elements are moved which are in contact to each other (e.g. pumps). The removal of aggressive solvents (e.g. acetonitril) is firstly advantegeous for the environment and secondly it prevents chemically unstable materials (e.g. rubber sealings) from breakedown. The removal of stationary phases for separation is advantouge to reduce overall costs due to clogging and destruction of those stationary phases. The time for changing conditions in traditionally HPLC is very time consuming which gets vanished if only gases or electric fields need to be changed.

The clinical diagnostic system may be performed in the multiple raction mode.

The ion detection station may be a charge detector or an optical detector.

The charge detector may refer to a semiconductor which can recognize a chared molecule by applying a voltage.

The optical detector may refer to a semiconductor which can recognize a photon by applying a voltage.

The clinical diagnostic system may comprise a data processing station. The data processing station may be for processing and/or evaluation of at least one electronic signal received at least from the ion detection system.

The data processing station may be computer based. Computer based can mean in this context that a computer is used or processing and/or evaluation and/or showing the at least one electronic signal or the information, the user wanted to know.

The data evaluation, i.e. identification and possibly quantitation and/or qualification of each analyte of interest for each sample can be made in the data processing station.

The analyte of interest may pass through the sample preparation station, then the ion generation station, then the at least one selectivity enhancer station, then the ion detection station and then the data processing station.

The analyte of interest may pass through the ion transportation unit, then a first ion fragmentation unit, then the ion mobility unit, and then a second ion fragmentation unit.

The at least one analyte of interest may be present in the said clinical diagnostic system and/or in the mass spectrometer and/or in the ion mobility unit of less than 10 seconds, preferably less than 9 seconds or less than 8 seconds or less than 7 seconds or less than 6 seconds or less than 5 seconds or less than 4 seconds or less than 3 seconds or less than 2 seconds or less than 1 second.

The method described herein may have the following features:

The internal standard may be added, preferably in step (A), wherein the internal standard may be preferably isotopically labelled.

The analyte of interest may be native or not native.

The method may be an in vitro method.

The method may be performed in a clinical diagnostic system according to the aspect of the present invention.

The biological sample may be prepared in a random-excess mode.

The biological sample may be obtained from a patient sample, which is selected from a group consisting of serum, plasma, tissue, liquor, breath, sweat, sputum and whole blood sample from an individual, preferably wherein the individual is a mammal, preferably a human.

The at least one analyte of interest may have a molar mass of smaller than m/z= 20 000, preferably smaller than m/z =10 000.

The sample preparation station may be configuredfor removing or at least reducing interfering matrix components in the biological sample and/or enriching analytes of interest in the biological sample and may comprise at least one of the following units or combinations thereof: sample delivery unit, sample pre-treatment unit, sample analytical unit.

The sample delivery unit may comprise sample collection and preparation and/or transportation, and/or wherein the sample pre-treatment unit may comprise at least one unit or more than one units selected from the following group: magnetic bead handling unit, internal standard handling unit, supporting compound handling unit, pipetting unit, fluid transport unit, reaction container transporting mechanism unit, enrichment handling unit, solvent evaporation unit, derivatization unit and/or wherein the sample analytical unit comprises at least one unit or more than one units selected from the group: a solid-liquid support analyte enrichment unit, solid-liquid support matrix depletion unit, gaseous sample enrichment or matrix separation unit, solid sample on solid support unit.

The sample preparation station may be partially or fully automated.

The ion generation station may comprise a steady analyte ion supply unit, wherein the steady analyte ion supply unit may be selected from the group consisting of flow injection (preferably flow injection using ESI or APCI or Nano Spray), paper spray, electron ionization (EI), matrix assisted ionization (MAI), chemical ionization (CI), e.g. proton-transfer-reaction (PTR), radioactive supported ionization, e.g. using ⁶³Ni and combinations thereof.

The selectivity enhancer station may comprise the ion mobility unit and at least one unit selected from the following group: ion transportation unit, ion fragmentation unit, wherein the ion transportation unit is selected from the group consisting of ion funnels, step wave, S-lens, ion mirror, ion fight tube by differential potential, ion trap, multipole, e.g. quadrupole, non-contact ion transport system, e.g. direct source coupling (e.g. heated capillary) and magnetic sector transport, wherein the ion fragmentation unit is selected from the group consisting of collision-induced dissociation (CID), electron-capture dissociation (ECD), electron-transfer dissociation (ETD), photon dissociation, ion trap, orbitrap, time-of-flight (TOF), magnetic sector, e.g. double focussion sector field (DFS) and combinations thereof.

The at least one analyte of interest may be present in the said clinical diagnostic system of less than 10 seconds, preferably less than 9 seconds or 8 seconds or 7 seconds or 6 seconds or 5 seconds or 4 seconds or 4 seconds or 3 seconds or 2 seconds or 1 second.

The clinical diagnostic system may be free of paper spray or Solid Phase Extraction (SPE). Preferably, the ion generation station for generating at least one ion or ions of the at least one analyte of interest may be free of or does not comprise paper spray or SPE.

The clinical diagnostic system may be free of an ion mobility separator comprising at least one coaxial mobility cell followed by conical coaxial ion channel.

The ion mobility unit may comprise a printed circuit board, preferably a stack of printed circuit boards.

The ion mobility unit may be free of a printed circuit board, preferably free of a stack of printed circuit boards.

The clinical diagnostic system may not comprise an ion source, preferably said source being filled with gas at gas pressure from generally at or between about 1 mBar to 1Bar;
an ion gate may be formed of a front cap electrode, followed by a front mesh and then by a back mesh, wherein the meshes are generally parallel with one another and spaced at a distance comparable to mesh cell size;
a radiofrequency (RF) generator may be connected between the meshes;
a switching or adjustable DC signal may be connected to the cap electrode and meshes;
an ion drift space, preferably filled with gas at pressure from generally at or between about (1 to 30) mBar; and an ion detector.

The clinical diagnostic system may be free of gas chromatography (GC).

The clinical diagnostic system may not separate positively charged ions and negatively charged ions in the gas phase.

The clinical diagnostic system may not comprise an annular electrode.

The clinical diagnostic system may not comprise an ion drift space, that preferably is filled with gas at pressure from substantially at or between about (1 to 100) mBar.

The clinical diagnostic system may not comprise an ion drift tube.

In embodiments of the method, the internal standard may be added, preferably in step (A), wherein the internal standard is preferably isotopically labelled.

In embodiments of the method, the said method may be performed in a clinical diagnostic system according to the aspect of the present invention.

### Examples

The following examples are provided to illustrate, but not to limit the presently claimed invention.

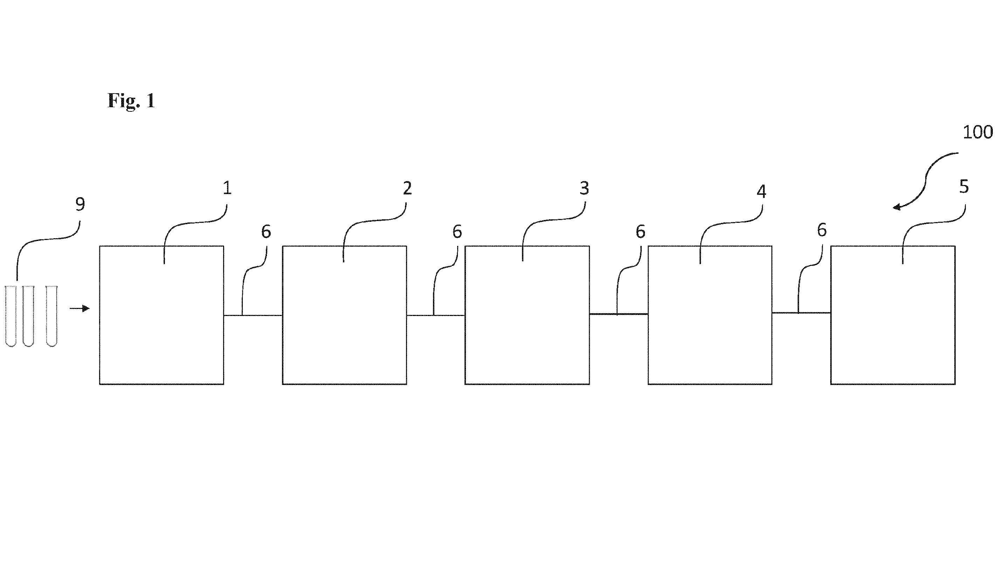

**Figure** 1 shows an example of a schematic representation of a clinical diagnostic system 100 and a clinical diagnostic method. The clinical diagnostic system 100 comprises a sample preparation station 1 for the automated preparation of biological samples 9 comprising at least one analyte of interest. The sample preparation station 1 is directly coupled 6 to the ion generation station 2 for generating at least one ion or ions of the at least one analyte of interest. The ion generation station 2 is directly coupled 6 to the selectivity enhancer station 3. The selectivity enhancer station 3 is for enhancing the selectivity of the at least one analyte of interest. The selectivity enhancer station 3 separates ions based on the respective ion size comprises an ion mobility unit 31 for separation ions with respect to their ion mobility using electric field and/or radio frequency fields (AC/DC and/or RF) applied to one or more electrodes. The selectivity enhancer station 3 is directly coupled 6 to the ion detection station 4 for detecting of at least one ion of the at least one analyte of interest. The ion detection station 4 is directly coupled 6 to the data processing station 5 for processing and/or evaluation of at least one electronic signal received at least from the ion detection station 4.

Preferably, the clinical diagnostic system 100 is automated and/or in a random-access mode.

Peferably, the clinical diagnostic system 100 is arranged according to the following embodiments E:

**Table 1:**

| E | Arrangement of the clinical diagnostic system 100 |
|---|---|
| E1 | 11,12,122,131,221,32,31,331,327,46,5 |
| E2 | 11, 12,122,132,221,32,31,331,327,46,5 |
| E3 | 11,12,122,123,134,128,211,31,321,331,324,337,42,5 |
| E4 | 11,12,122,123,134,128,224,31,321,331,324,337,42,5 |
| E5 | 11,12,133,223,31,321,331,324,337,45,5 |
| E6 | 11,12,122,121,222,323,333,326,31,327,42,5 |
| E7 | 11,12,122,128,213,323,326,328,331,328,31,326,331,41,5 |

| | |
|---|---|
| E - embodiment, e.g. E1 - embodiment 1 | |

The numbers mentioned in the table 1 represent the references of the respective stations and/or units mentioned in the list of references.

The numbers are separated by commas, which means that adjacent numbers separating by commas are adjacent stations and/or units, which are coupled. For example:

### embodiment 1 (E1)

The clinical diagnostic system 100 of embodiment 1 (E1) has the following arrangement:
11,12,122,131,221,32,31,331,327,46,5

The sample delivery unit 11, then sample pre-treatment unit 12, then internal standard handling unit 122, then solid-liquid support analyte enrichment unit 131, then flow injection (preferably flow injection using ESI or APCI or Nano Spray) 221, then ion transportation unit 32, then ion mobility unit 31, then collision-induced dissociation (CID) 331, then multipole, e.g. quadrupole 327, then electron multiplier 46 and then data processing station 5.

The advantage of E1 and/or E2 is that the separation of the analytes and therefore the matrix reduction and isobaric separation not only performed by the workflow itself (e.g. magnetic microparticles and or liquid extraction). The ion mobility unit 31 performs as a selectivity enhancer. The task for this enhancer has been made in previously comparable workflows (e.g. with HPLC for isobaric separation and matrix reduction) was done. Furtheremore to add the ion mobility unit 31 in these workflows and substitute HPLC is furthermore advantageous due to the fact that the ion mobility unit 31 will compress the analyte signal time dependently into one peak-shaped signal and therefore further enriches the analyte time dependend.

The advantage of E3 and/or E4 is that for a solid supported analysis from a spot on a surface, when an HPLC separation is not applicale and therefore the ion mobility unit is adding value due to the possibility of having isobaric separation and matrix depletion in ion gas phase.

The advantage of E5 is that the temperature dependend separation of GC (gas chromatography) can be vanished by having the ion mobility unit 31. Therefore no oven for heating or cooling is required to perform the isobaric separation of unwanted matrix components.

The advantage of E6 is that a direct sample analysis on a paper tissue by paper spray ionization can vanish any active zones for matrix reduction or analyte enrichment on the respective paper strip. The separation will be performed therefore with the ion mobility unit 31.

The advantage of E7 is that a surface ionization with a plasma ionization can be used directly on a tissue or a living organism. This surface ionization can be used directly as a cold plasma which not damages living system but ionizes softly the surface molecules which can be further analyzed in real time by mass spectrometry e.g. during a surgery or on cell suspensions.

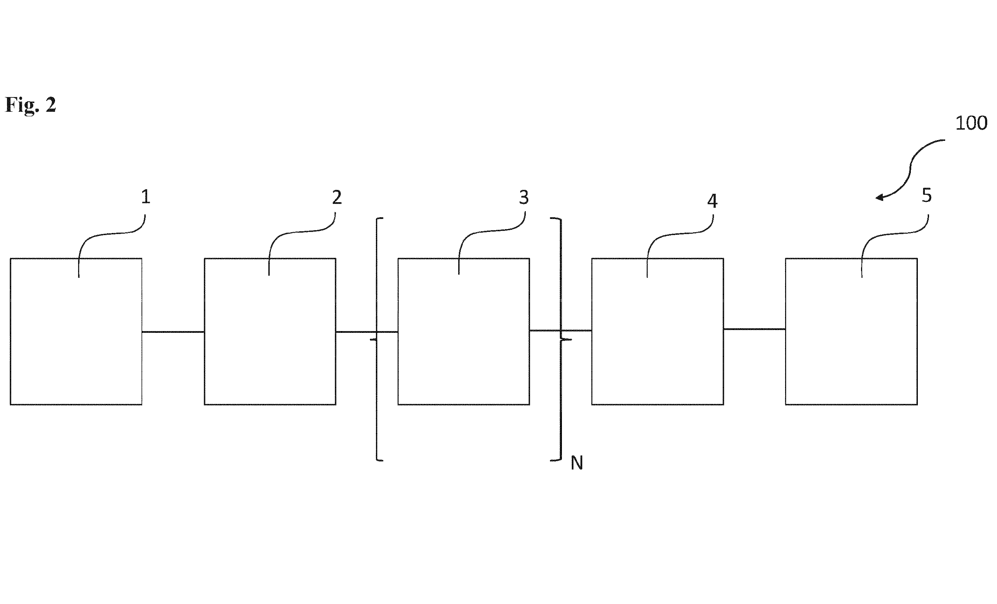

**Figure** 2 shows an example of a schematic representation of a clinical diagnostic system 100 and a clinical diagnostic method. In this example of Figure 2 it is shown compared to the clinical diagnostic system 100 of Figure 1 that the selectivity enhancer station 3 can be N-times part of the clinical diagnostic system 100, where N is e.g. 2 or 3 or 4 or 5 or 6. Thus, the enhancement of the selectivity of the at least one analyte of interest can be increased. The advantage of this system is the combination of different fragmentation mechanisms e.g. CID with ETD and in between selection of certain fragments by their respective ion mobility. The selectivity of the system is therefore enhanced if multiple selectivity stations are made through.

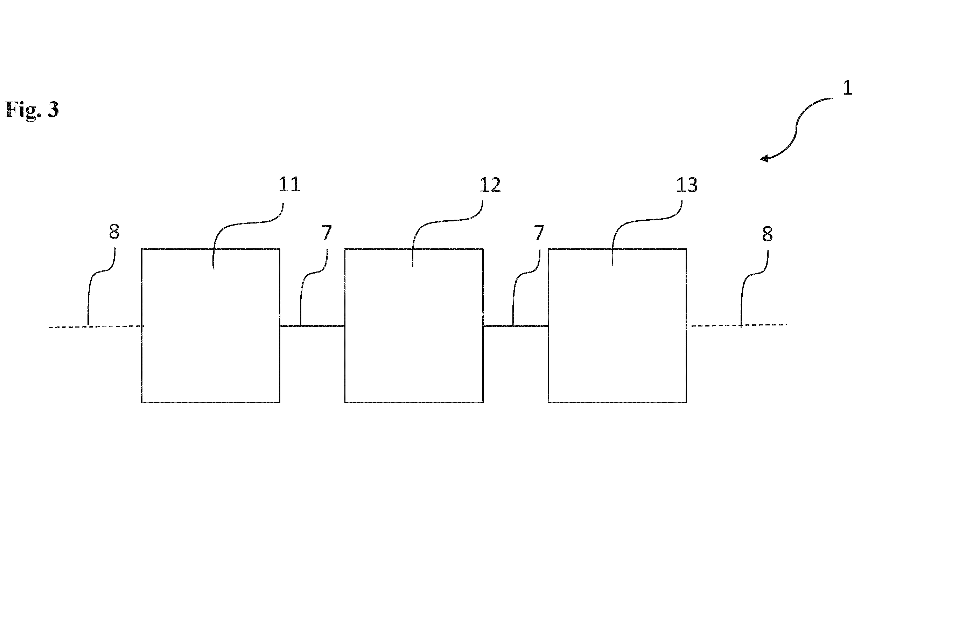

**Figure** 3 shows an example of a schematic representation of a sample preparation station 1. The sample preparation station 1 comprises a sample delivery unit 11, a sample pre-treatment unit 12 and a sample analytical unit 13. The sample pre-treatment unit 12 is arranged between the sample delivery unit 11 and the sample analytical unit 13. The sample analytical unit 13 and optionally the sample preparation station 1 can be coupled to adjacent stations and/or units 8. The sample delivery unit 11 comprises sample collection and preparation, e.g. including sample data preparation and/or transportation. In this station the sample is prepared to get addressed by a pipettor e.g. arrended horizontically etc. followed by meta data reading (e.g. barcode reader) followd by sorting if necessary as well as heating or cooling or shaking. The sample pre-treatment unit 12 is for preparing the analyte for quantitative analyses e.g. pipetting of internal standard or adjustment of the pH value. Further binding protein or protein precipitation can be achieved by adding supporting reagents. A heating/cooling or evaporation step can be performed in the sample preparation unit. The sample pre-treatment unit 12 comprises at least one unit or more than one units selected from the following group: magnetic bead handling unit 121, internal standard handling unit 122, supporting compound handling unit 123, pipetting unit 124, fluid transport unit 125, reaction container transporting mechanism unit 126, enrichment handling unit 127, solvent evaporation unit 128. Adjacent to the sample pre-treatment unit 12, the sample analytical unit 13 is arranged. The sample analytical unit 13 is for generation of an ion from the target molecule of interest (analyte). Further the separation of this analyte from unwanted matrix and enhancement of the selectivity by either m/z values or ion mobility. The detection of the selectively enhanced analyte is done by detection of the ion. The sample analytical unit 13 comprises at least one unit or more than one units or a combination of the units selected from the group: a solid-liquid support analyte enrichment unit 131, solid-liquid support matrix depletion unit 132, gaseous sample enrichment or matrix separation unit 133, solid sample on solid support unit 134.

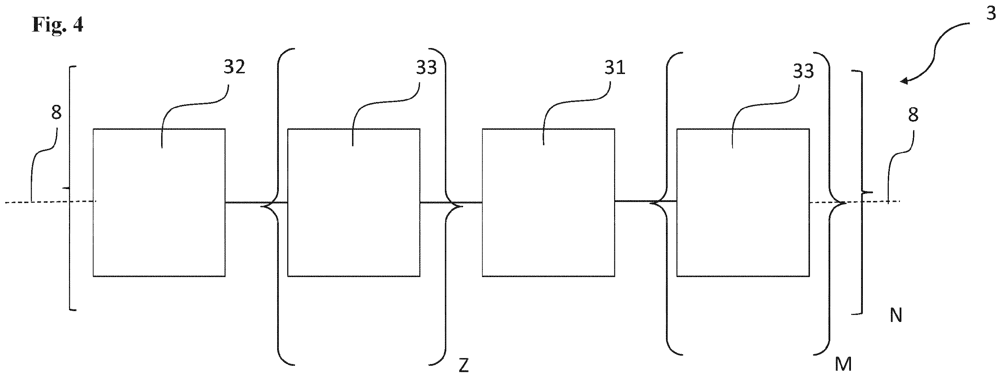

**Figure 4** shows an example of a schematic representation of at least one selectivity enhancer station 3. If N = 1, it is exactly one selectivity enhancer station 3 shown. If N = 2 or 3, then two or three selectivity enhancer stations are examplied shown. The selectivity enhancer stations may have the same or different units and are coupled adjacent to each other. The at least one selectivity enhancer station 3 separates ions based on the respective ion size. The at least one selectivity enhancer station comprises an ion mobility unit 31 for separation ions with respect to their ion mobility using electric and/or radio frequency fields (AC/DC and/or RF) applied to one or more electrodes. The ion mobility unit 31 is arranged directly between two ion fragmentation units 33 (if M and Z > 0) or directly between the ion transportation unit 32 (if Z = 0) and the ion detection station 4 (if M = 0). The ion transportation unit 32 is selected from the group consisting of ion funnels, step wave, S-lens, ion mirror, ion fight tube by differential potential, ion trap, multipole, e.g. quadrupole, non-contact ion transport system, and magnetic sector transport. The ion fragmentation unit 33 is selected from the group consisting of collision-induced dissociation (CID), electron-capture dissociation (ECD), electron-transfer dissociation (ETD), photon dissociation, ion trap, orbitrap, time-of-flight (TOF), magnetic sector, e.g. double focussion sector field (DFS).

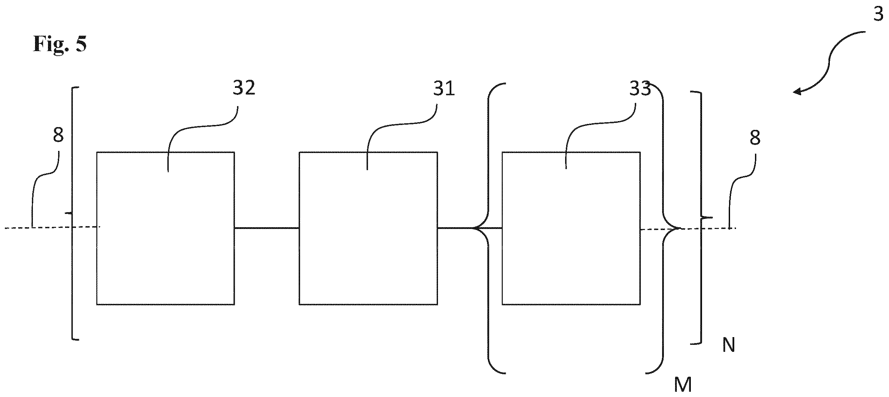

**Figure 5** shows an example of a schematic representation of at least one selectivity enhancer station 3. Here, the ion mobility unit 31 is arranged directly between the ion transportation unit 32 and at least one ion fragmentation unit 33 (if M > 0) or ion detection station 8, 4 (if M = 0).

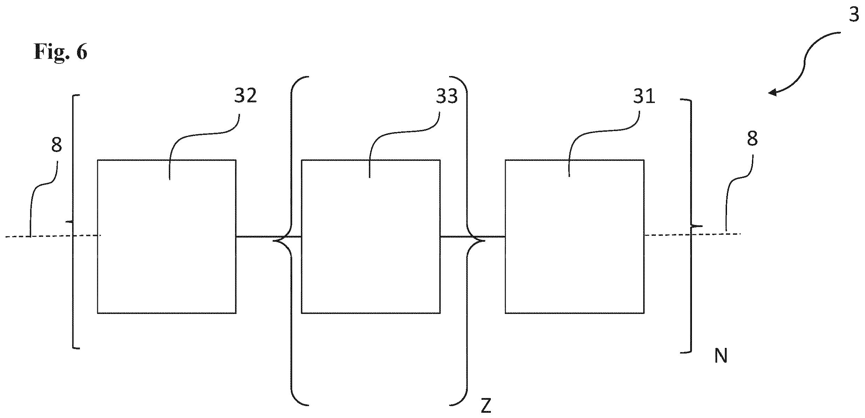

**Figure 6** shows an example of a schematic representation of at least one selectivity enhancer station 3. Here, the ion mobility unit 31 is arranged directly between the ion transportation unit 32 (if Z = 0) or at least one ion fragmentation unit 33 (if Z > 0) and the ion detection station 8, 4. The at least one ion fragmentation unit 33 can be arranged between the ion mobility unit 31 and the ion transportation unit 32.

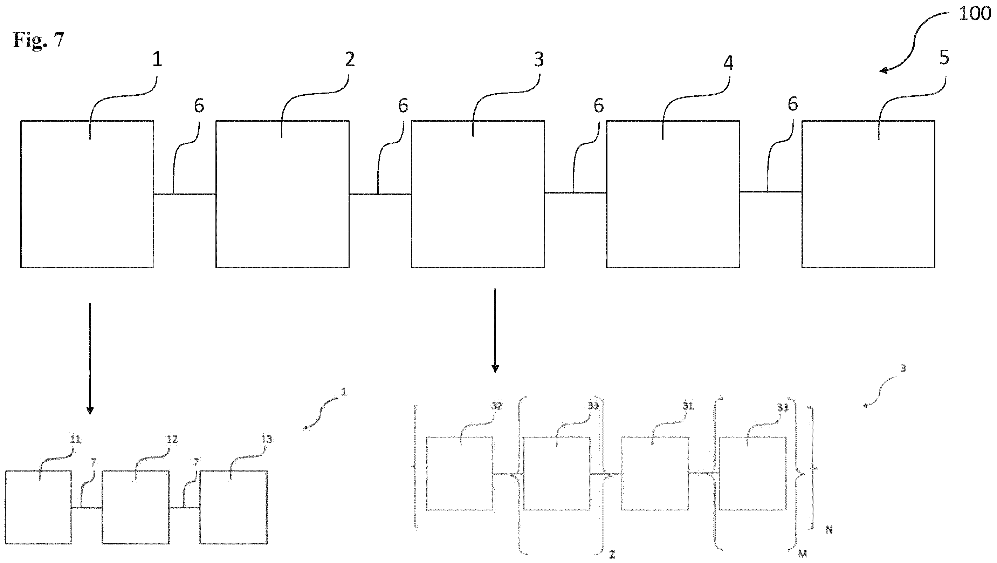

**Figure 7** shows an example of a schematic representation of a clinical diagnostic system 100 and a clinical diagnostic method. This clinical diagnostic system 100 of Figure 7 shows in comparision to the clinical diagnostic system 100 of Figure 1 the units of the sample preparation station 1 (sample delivery unit 11, sample pre-treatment unit 12, sample analytical unit 13) and the units of the selectivity enhancer station 3 as explained in Figure 4.

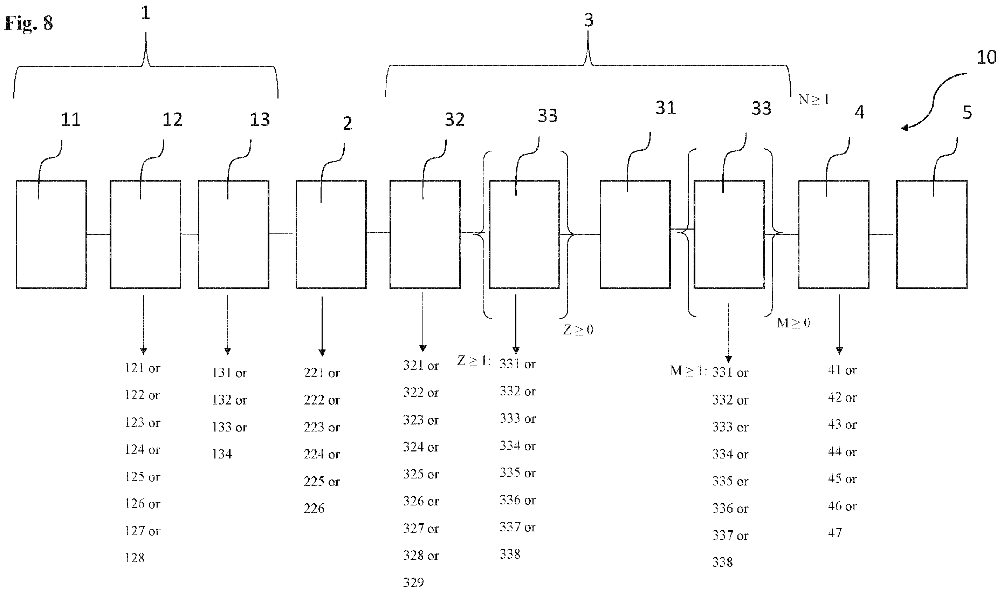

**Figure 8** shows an example of a schematic representation of a clinical diagnostic system 100 and a clinical diagnostic method. Here, the arrangement of the respective stations and units is shown.

Preferably, the following embodiments A to C a clinical diagnostic system 100 are described:
**A) Direct ionization of the sample after sample preparation with nano ESI**
   The clinical diagnostic system 100 consists of or comprises the following units and/or stations: 11,12,121,122,123,124,13,221,323,31,322,327,331,327,43,5
   The sample enters the workflow by being a human body fluid which is further processed by pipetting internal standard, followed by a magnetic bead workflow, multiple solid liquid separation and washing steps supported by magnetic forces. After the final washing step the analyte loaded magnetic particles gets eluted by a supporting liquid followed by a direct nano-ESI ionization and ion transport by a S-lens to the ion mobility unit which separats isobaric components off and passes through the analyte of interest which gets m/z separated by the first quadrupole, fragmented by CID and the fragment separated by m/z with a second quadrupole. An ion detector detects the analyte and the internal standard signal and processes those signals with a computer to finally end with absolute quantitative results. The time for the outside of the mass spectrometer is in this workflow about 10s-1000s and inside the mass spectrometer about 0.1s-10s.
B) Ionization via matrix assisted laser ionization
   The clinical diagnostic system 100 consists of or comprises the following units and/or stations: 11,12,121,122,123,124,128,134,211,322,31,322,327,331,327,43,5
   The sample enters the workflow by being a human body fluid which is further processed by pipetting internal standard, followed by a magnetic bead workflow, multiple solid liquid separation and washing steps supported by magnetic forces. After the final washing step, the analyte loaded magnetic particles gets eluted by a supporting liquid followed by pipetting and drying onto a LDI target followed by laser irradiation and guiding into the mass spectrometer via an ion funnel to the ion mobility unit which separats isobaric components off and passes through the analyte of interest which gets m/z separated by the first quadrupole, fragmented by CID and the fragment separated by m/z with a second quadrupole. An ion detector detects the analyte and the internal standard signal and processes those signals with a computer to finally end with absolute quantitative results. The time for the outside of the mass spectrometer is in this workflow about 10s-1000s and inside the mass spectrometer about 0.1s-10s.
**C) Ionization via matrix assited ionization**
   The clinical diagnostic system 100 consists of or comprises the following units and/or stations: 11,12,121,122,123,124,128,134,224,328,31,322,327,331,327,43,5
   The sample enters the workflow by being a human body fluid which is further processed by pipetting internal standard, followed by a magnetic bead workflow, multiple solid liquid separation and washing steps supported by magnetic forces. After the final washing step the analyte loaded magnetic particles gets eluted by a supporting liquid and addition of a supporting solid e.g. matrix compound followed by pipetting and drying onto a glass target followed by non-contact ion transport system (328, direct ion contacting unit) e.g. heated capillary and guiding into the mass spectrometer which separats isobaric components off and passes through the analyte of interest which gets m/z separated by the first quadrupole, fragmented by CID and the fragment separated by m/z with a second quadrupole. An ion detector detects the analyte and the internal standard signal and processes those signals with a computer to finally end with absolute quantitative results.
   The time for the outside of the mass spectrometer is in this workflow about 10s-1000s and inside the mass spectrometer about 0.1s-10s.

With continued reference to Figure 1 a clinical diagnostic method is also illustrated. The method comprises automatically preparing biological samples 9 comprising analytes of interest and inputting prepared samples into ion generation station 2 for generating at least one ion or ions of the at least one analyte of interest, which are gaseous. Then, the ion or ions are separated via their ion mobility using an electric field applied to one or more electrodes in the ion mobility unit 31. Then, the at least one ion of the at least one analyte of interest is detected or determined using mass spectrometry. Peferably the clinical diagnostic method and the clinical diagnostic system 100 is free of a chromatography unit, preferably a liquid chromatography (LC) unit or a HPLC unit.

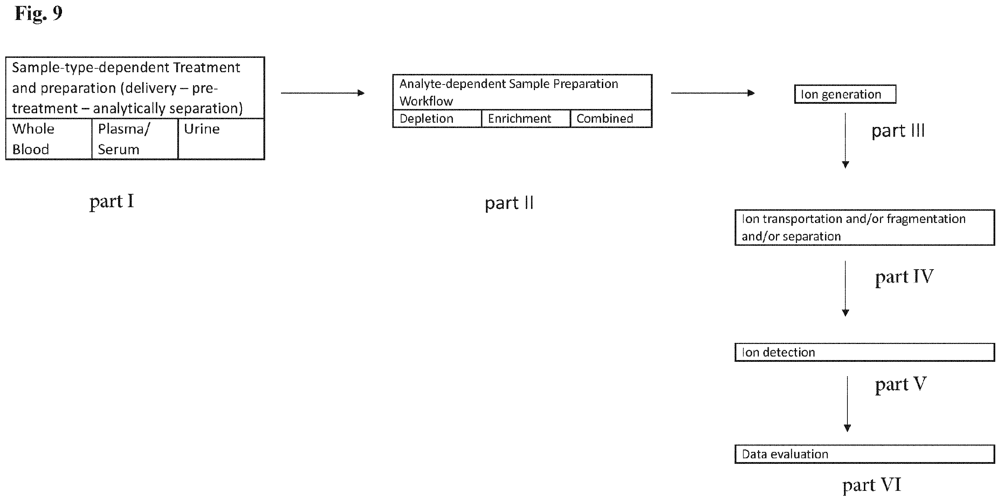

**Figure** 9 schematically represents elements of a clinical diagnostic method and in particular a combination of possible workflow paths, depending on the sample type and/or the analytes of interest in a sample.

For example, depending on the type of sample, e.g. whole blood, plasma, serum, urine, a sample can be assigned to one of some pre-defined sample type-dependent pre-treatment workflows (part I) that is most appropriate for that type of sample.

Also, depending on the analytes of interest, e.g. individual analytes or classes of analytes having similar chemical structure or properties, following the sample-type dependent (pre-)treatment in part I, a sample can be assigned to one of some pre-defined analyte-dependent sample preparation workflows, e.g. based on a matrix depletion method, an analyte enrichment method, or a combination of both, that is most appropriate for that type of analyte(s) (part II).

The analytes of interest, a prepared sample can be assigned to a particular ion generation station 2 for generation ions, preferably in gaseous form (part III).

Also, depending on the analytes of interest, a prepared sample can be assigned to a particular selectivity enhancer station 3 for separating or transferring that type of analyte(s) to the ion detection station 4 (parts IV and V).

Finally, following mass spectrometry, data evaluation (part VI), i.e. identification and possibly quantitation of each analyte of interest for each sample can be made.

In particular, the method describes herein is perfomed in a random-access mode, with a high-throughput MS set up, wherein several different analytes exhibiting different chemical properties have to be measured in a short amount of time.

High-throughput MS set up can mean that at least 10 samples per hour can be measured, preferably 100 samples /h or more than 100 samples per hour, e.g. 120 or 130 or 140 or 150 or 160 or 170 or 180 or 190 or 200 or 300 or 400 or 500 or 600 samples per hour.

Short amount of time can mean that a run for one sample is finished in 360s, preferably in excact 360s or more than 360 s.

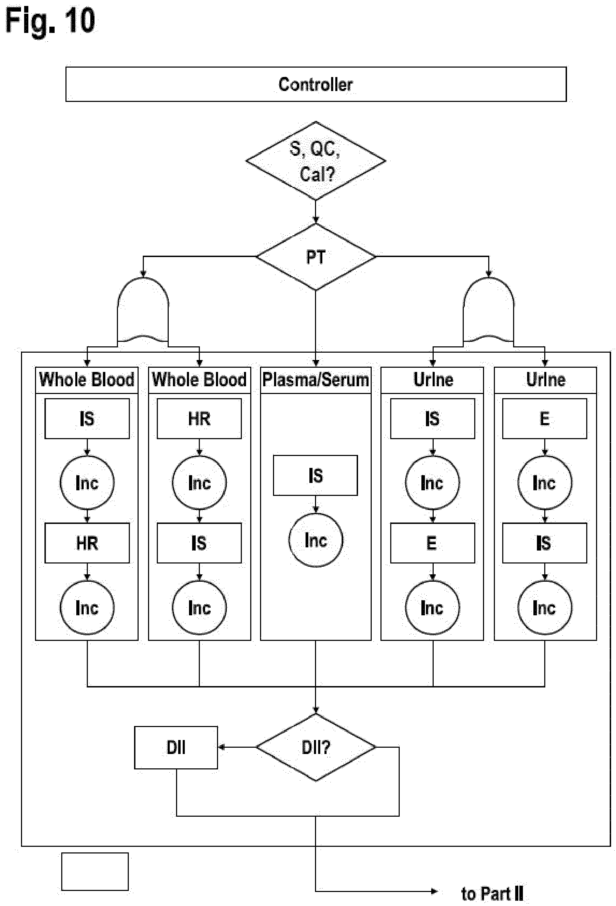

**Figure 10** is an examplified flow chart representing part I of the clinical diagnostic method of Figure 9 in more detail, and in particular the sample pre-treatment part. A controller first determines whether to assign a pre-defined workflow to a sample S or whether a quality control (QC) or a calibration (Cal) has to be carried out. A QC and/or a calibration workflow may follow at least some of the same steps as one of the sample workflows with the possible addition or deletion of some of the steps. For the purpose of this example only sample-type-dependent pre-treatment (PT) workflows are described.

For example, if the sample is a whole blood sample, it is assigned to one of two pre-defined sample PT workflows, both comprising the addition of an internal standard (IS) and a hemolysis reagent (HR) followed by a pre-defined incubation period (Inc), where the difference between the two workflows is the order in which the internal standard (IS) and a hemolysis reagent (HR) are added. An internal standard (IS) is typically a known amount of the same analyte(s) of interest that may be for example isotopically labeled. This allows relative comparison, and may enable unambiguous identification and quantification of the analyte(s) of interest present in the sample when the analyte(s) reach the mass spectrometer.

If the sample is a urine sample, it is assigned to one of other two pre-defined sample PT workflows, both comprising the addition of an internal standard (IS) and an enzymatic reagent (E) followed by a pre-defined incubation period (Inc), where the difference between the two workflows is the order in which the internal standard (IS) and a enzymatic reagent (HR) are added. An enzymatic reagent is typically a reagent used for glucuronide cleavage or protein cleavage or any preprocessing of analyte or matrix.

If the sample is plasma or serum it is assigned to another pre-defined PT workflow including only the addition of an internal standard (IS) followed by a pre-defined incubation time (Inc). Optionally, it may further include the addition of a lysis reagent (not shown).

All of the above sample-type-dependent PT workflows may comprise the addition of a dilution liquid (Dil). However, the addition of a dilution liquid (Dil) may be also specifically linked to any one or more of the above sample-type-dependent PT workflows.

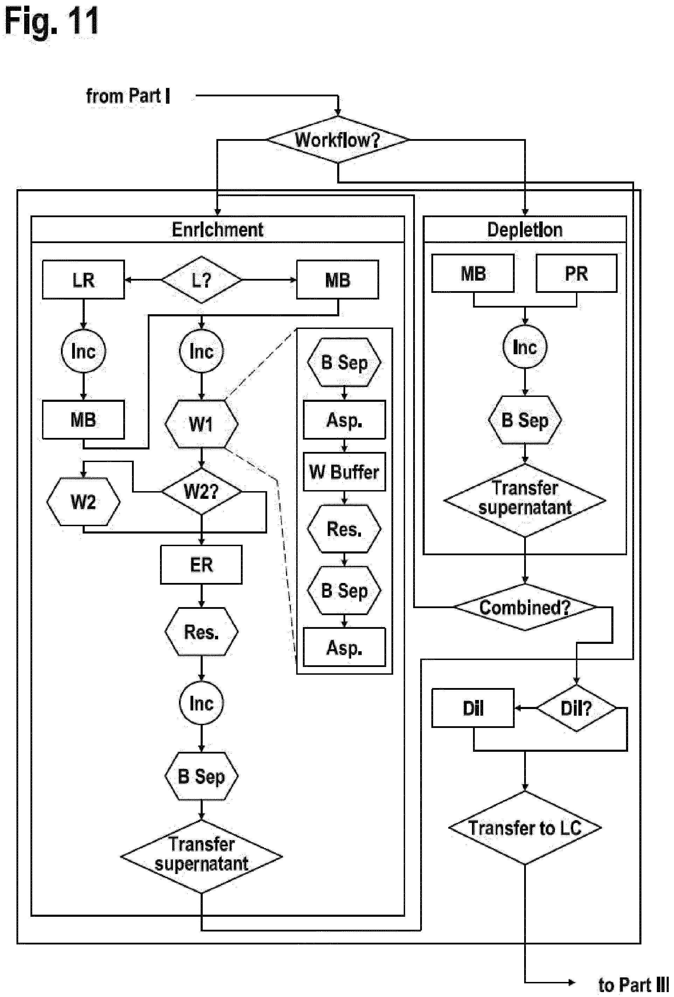

**Figure 11** is an examplified flow chart representing Part II of the clinical diagnostic method of Figure 9 in more detail and is a continuation of the flow chart of Figure 10. In particular, the controller assigns each pre-treated sample to one of pre-defined analyte-dependent sample preparation workflows depending on the analyte(s) of interest in the pre-treated sample.

For example, a pre-treated sample can undergo an analyte enrichment workflow, a matrix depletion workflow or a matrix depletion workflow followed by an analyte enrichment workflow.

The analyte enrichment workflow comprises addition of magnetic beads (MB) carrying analyte-selective groups to the pre-treated sample followed by a pre-defined incubation period (Inc) for capturing the analyte(s) of interest, where the addition of the magnetic beads (MB) may include agitation or mixing. The addition of the magnetic beads (MB), depending on the analyte(s) of interest, may be preceded by addition of a lysis reagent (LR) followed by a pre-defined incubation period (Inc). A lysis reagent (LR) can be a reagent for lysis of erythrocytes or release from binding protein or release from unspecific binding. After incubation with the magnetic beads (MB) the workflow comprises a washing step (W1) and depending on the analyte(s) possibly one or more additional washing steps (W2). A washing step (W1, W2) comprises a series of steps including magnetic bead separation (B sep) by a magnetic bead handling unit comprising magnets or electromagnets, aspiration of liquid (Asp.), addition of a washing buffer (W. Buffer), resuspension of the magentic beads (Res.), another magnetic bead separation step (B Sep) and another aspiration of the liquid (Asp.). Moreover washing steps may differ in terms of type of solvent (water/organic/salt/pH), apart from volume and number or combination of washing cycles.

The last washing step (W1, W2) is followed by the addition of an elution reagent (ER) followed by resuspension (Res.) of the magnetic beads and a pre-defined incubation period (Inc.) for releasing the analyte(s) of interest from the magnetic beads. The bound-free magnetic beads are then separated (B Sep.) and the supernatant containing the analyte(s) of interest can be directly transferred to the LC station or after a dilution step by addition of a dilution liquid (Dil). Different elution procedures/reagents may also be used, by changing e.g. the type of solvents (water/organic/salt/pH) and volume.

The matrix depletion workflow comprises the addition of magnetic beads carrying matrix selective groups (MB) and a precipitation reagent (PR) to the pre-treated sample followed by a pre-defined incubation period (Inc.), for capturing the matrix components while leaving the analyte(s) of interest in the supernatant, followed by Bead separation (B Sep.) and transfer of the supernatant containing the analyte(s) of interest to LC station directly or after dilution (Dil).

Depending on the analyte(s) of interest, the supernatant derived from the matrix depletion workflow can undergo the analyte enrichment workflow following thereby a combined sample preparation workflow.

Parts III to VI can be continued after the examplified flow chart representing Parts I and II of the clinical diagnostic method of Figures 10 and 11. Additionally, a Part VII can be followed, which can be a computer unit for controlling the AC/DC and RF values, gas flows and/or temperatures of the certain units. The controlling is bidirectional controlled by readbacks of AC/DC and RF values, gas flows and/or temperatures of the certain units. The controlling can be triggerd within ms (milliseconds) up to hours depending on the run time of the dertain unit.

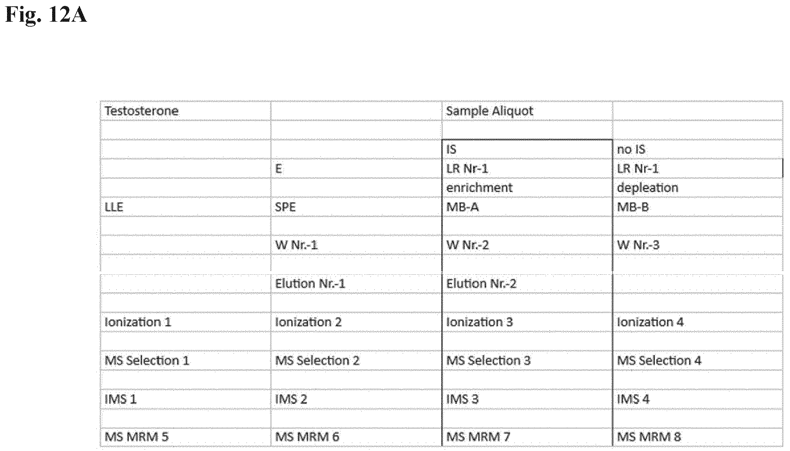

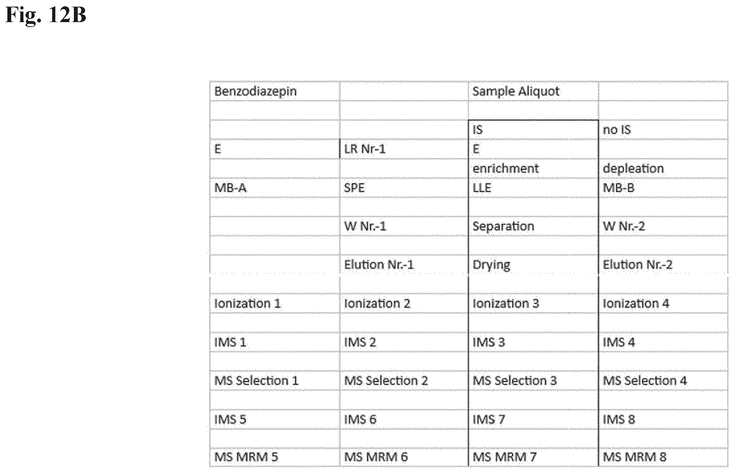

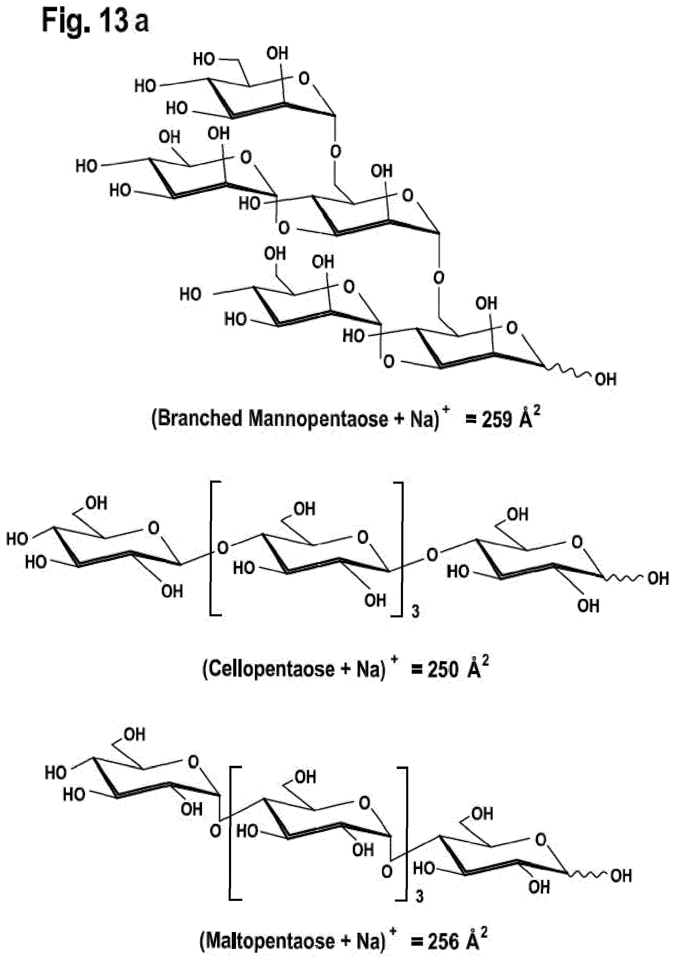

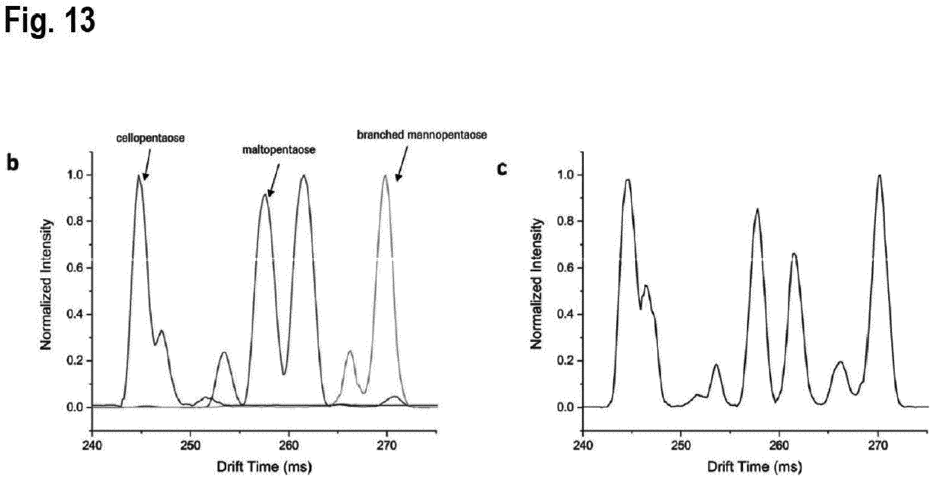

**Figures 12A** **and** **12B** provide two generic examples of analyte specific workflow paths that may be pre-defined in a master table or memory, each including a selection of options among generally selectable options (only most relevant options are indicated for simplicity). These general selectable options are for example the addition of an internal standard (IS) or no addition of IS (no IS); the addition of an enzymatic reagent (E) or one of two lysis reagents (LR #1, LR#2); the enrichment or depletion workflow; one type of magnetic beads (MB A, MB B, MB C, MB D) where different types of magnetic beads selective for groups of analytes (eg. polar, unpolar, charged, uncharged) or specific for selected analytes (specific binders); other types of sample pre enrichment like LLE (liquid-liquid extraction) or SPE (soplid phase extraction) are possible one of different pre-defined washing procedures (W Nr.-1, W Nr.-2, W Nr.-3, W Nr.-4) including different types of washing liquid (water/organic/salt/pH), volume, number or combination of washing cycles; type of Ionization (Ionization 1, Ionization 2, Ionization 3, Ionization 4, e.g. MAI, Paper Spray, MALDi, direct infusion ESI, nanao ESI, etc.); one of different pre-defined elution procedures (elution Nr.-1, elution Nr.-2, elution Nr.-3, elution Nr.-4), including different types of elution liquid (water/organic/salt/pH) or volume; type of ion mobility (with or without supporting gas in differnet AC/DC and RF and gas configurations); one of different pre-defined mass spectrometric acquisition procedures (settings for ionization, ion transfer, ion selection and fragmentation, detection and data acquisition) and shortly summarized as MS MRM 5, MS MRM 6, MS MRM 7, MS MRM 8, MS Selection by pre filtering e.g with ion trap, or multipole e.g. ion guide. The ion mobility is shortly summarized as IMS 1, IMS 2, IMS 3, IMS 4.

If the analyte of interest is for example testosterone, the testosterone-specific workflow includes the addition of an internal standard (IS), the addition of a lysis reagent (LR Nr.-1), an enrichment workflow, the addition of testosterone selective magnetic beads (MB A), one of the pre-defined washing procedure (W Nr.-2), one of the pre-defined elution procedure (elution Nr.-2), ionization 3 by nano-ESI, the use MS Selection Nr. 3 (Ion Trap) and Ion Mobility Nr. 3, and one of the pre-defined mass spectrometric acquisition procedures (MS MRM 7).

If the analytes of interest are for example benzodiazepines, the benzodiazepine-specific workflow includes the addition of an internal standard (IS), the addition of an enzymatic reagent (E), an enrichment workflow, the addition non mixable solvent to the sample media e.g. ethylacetate, liquid separation stept by pipetting, drying step on a surface for SALDI, SALDI ionization, MS Selection by a multipole, IMS and one of the pre-defined mass spectrometric acquisition procedures (MS MRM 7).

Similarly, workflow paths may be pre-defined for any sample/analyte of interest or groups of analytes of interest.

Modifications and variations of the disclosed embodiments are certainly possible in light of the above description. It is therefore to be understood, that within the scope of the appended claims, the invention may be practiced otherwise than as specifically devised in the above examples.

### List of References

100 - clinical diagnostic system
1 - sample preparation station
   11 - sample delivery unit
   12 - sample pre-treatment unit
      121 - magnetic bead handling unit
      122 - internal standard handling unit
      123 - supporting compound handling unit
      124 - pipetting unit
      125 - fluid transport unit
      126 - reaction container transporting mechanism unit
      127 -enrichment handling unit
      128 - solvent evaporation unit
   13 - sample analytical unit
      131 - solid-liquid support analyte enrichment unit
      132 - solid-liquid support matrix depletion unit
      133 - gaseous sample enrichment or matrix separation unit
      134 - solid sample on solid support unit
2- ion generation station
   21 - unsteady analyte ion supply unit
      211 - laser desorption ionzation (LDI)
      212 - dielectric barrier liquid flow
      213 - surface plasma ionization
   22 - steady analyte ion supply unit
      221 - flow injection (preferably flow injection using ESI or APCI or Nano Spray)
      222 - paper spray
      223 - electron ionization (EI)
      224 - matrix assisted ionization (MAI)
      225 - chemical ionization (CI), e.g. proton-transfer-reaction (PTR)
      226 - radioactive supported ionization, e.g. using ⁶³Ni.
3 - at least one selectivity enhancer station
   31 - ion mobility unit
   32 - ion transportation unit
      321 - ion funnels
      322 - step wave
      323 - S-lens
      324 - ion mirror
      325 - ion fight tube by differential potential
      326 - ion trap
      327 - multipole, e.g. quadrupole
      328 - non-contact ion transport system (e.g. heated capillary)
      329 - magnetic sector transport
   33 - at least one ion fragmentation unit
      331 - collision-induced dissociation (CID)
      332 - electron-capture dissociation (ECD)
      333 - electron-transfer dissociation (ETD)
      334 - photon dissociation
      335 - ion trap
      336 - orbitrap
      337 - time-of-flight (TOF)
      338 - magnetic sector, e.g. double focussion sector field (DFS)
4 - ion detection station
   41 - faraday cup
   42 - microchannel plate detector (MCP)
   43 - photomultiplier
   45 - orbitrap
   46 - electron multiplier
   47 - photographic plate
5 - data processing station
6 - coupling between the stations
7 - coupling between the units
8 - coupling to the adjacent stations and/or units
9 - sample or biological sample or samples or biological samples
N - number of selectivity enhancer stations, where N ≥ 1, e.g. 1 or 2 or 3 or 4 or 5 or 6
M - number of ion fragmentation unit, where M ≥ 0
Z - number of ion fragmentation unit, where M ≥ 0

## Claims

1. An automated clinical diagnostic system (100) for determining the presence or the level of at least one analyte of interest in a biological sample comprising
- a sample preparation station (1) for the automated preparation of biological samples (9) comprising at least one analyte of interest,
- an ion generation station (2) for generating at least one ion or ions of the at least one analyte of interest, wherein the ion generation station (2) comprises a steady analyte ion supply unit comprising a flow injection that uses electrospray ionization;
- at least one selectivity enhancer station (3) or more for enhancing the selectivity of the at least one analyte of interest, wherein the at least one selectivity enhancer station (3) is configured to separate ions based on the respective ion size and comprises an ion mobility unit (31) for separation of ions with respect to their ion mobility using electric and/or radio frequency fields (AC/DC and/or RF) applied to one or more electrodes, wherein the ion mobility unit (31) is free of a stack of printed circuit boards,
- an ion detection station (4) for detecting of at least one ion of the at least one analyte of interest, using mass spectrometry, and
- a data processing station (5) for processing and/or evaluation of at least one electronic signal received at least from the ion detection system,
wherein the sample preparation station (1) is directly connected to the ion generation station (2), which is directly connected to the at least one selectivity enhancer station (3), which is directly connected to the ion detection station (4),
wherein the sample preparation station comprises a sample pre-treatment unit which comprises at least one unit or more than one units selected from the following group: magnetic bead handling unit, internal standard handling unit, supporting compound handling unit, pipetting unit, fluid transport unit, reaction container transporting mechanism unit, enrichment handling unit, solvent evaporation unit, derivatization unit, and
wherein the clinical diagnostic system (100) is free of a chromatography unit.

2. The clinical diagnostic system (100) of claim 1, wherein the biological sample (9) is prepared in a random-excess mode.

3. The clinical diagnostic system (100) of claim 1 or 2, wherein the biological sample (9) is obtained from a patient sample, which is selected from a group consisting of serum, plasma, tissue, liquor, breath, sweat, sputum and whole blood sample from an individual, preferably wherein the individual is a mammal, preferably a human.

4. The clinical diagnostic system (100) of any of the proceeding claims, wherein the at least one analyte of interest has a molar mass of smaller than m/z= 2000, preferably smaller than m/z =1000.

5. The clinical diagnostic system (100) of any of the proceeding claims, wherein the sample preparation station (1) is for removing or at least reducing interfering matrix components in the biological sample (9) and/or enriching analytes of interest in the biological sample (9) and comprises at least one of the following units or combinations thereof: sample delivery unit, sample analytical unit.

6. The clinical diagnostic system (100) of any of the proceeding claims, wherein the sample delivery unit comprises sample collection and preparation and/or transportation, and/or wherein the sample analytical unit comprises at least one unit or more than one units selected from the group: a solid-liquid support analyte enrichment unit, solid-liquid support matrix depletion unit, gaseous sample enrichment or matrix separation unit, solid sample on solid support unit.

7. The clinical diagnostic system (100) of any of the proceeding claims, wherein the sample preparation station (1) is partially or fully automated.

8. The clinical diagnostic system (100) of any of the proceeding claims, wherein the ion generation station (2) comprises a steady analyte ion supply unit (22), wherein the unsteady analyte ion supply unit (21) is selected from the group consisting of laser desorption ionzation (LDI, 211), dielectric barrier liquid flow (212), surface plasma ionization (213) and combinations thereof, wherein the steady analyte ion supply unit (22) is selected from the group consisting of flow injection (221), paper spray (222), electron ionization (EI, 223), matrix assisted ionization (MAI, 223), chemical ionization (CI, 225), e.g. proton-transfer-reaction (PTR), radioactive supported ionization (226), e.g. using ⁶³Ni and combinations thereof.

9. The clinical diagnostic system (100) of any of the proceeding claims, wherein the selectivity enhancer station (3) comprises the ion mobility unit (31) and at least one unit selected from the following group: ion transportation unit (32), ion fragmentation unit (33), wherein the ion transportation unit (32) is selected from the group consisting of ion funnels (321), step wave (322), S-lens (323), ion mirror (324), ion fight tube by differential potential (325), ion trap (326), multipole (327), e.g. quadrupole, non-contact ion transport system (328), e.g. direct source coupling or heated capillary, and magnetic sector transport (329), wherein the ion fragmentation unit (33) is selected from the group consisting of collision-induced dissociation (CID, 331), electron-capture dissociation (ECD, 332), electron-transfer dissociation (ETD, 333), photon dissociation (334), ion trap (335), orbitrap (336), time-of-flight (TOF, 337), magnetic sector (338), e.g. double focussion sector field (DFS) and combinations thereof.

10. The clinical diagnostic system (100) of any of the proceeding claims, wherein the at least one analyte of interest is present in the said clinical diagnostic system (100) or in the mass spectrometer or in the ion mobility unit (31) of less than 10 seconds, preferably less than 9 seconds or less than 8 seconds or less than 7 seconds or less than 6 seconds or less than 5 seconds or less than 4 seconds or less than 3 seconds or less than 2 seconds or less than 1 second.

11. Use of the clinical diagnostic system (100) of any one of claims 1 to 10 for determining the presence or the level of the at least one analyte of interest in the biological sample (9).

12. An automated method for determining the presence or level of at least one analyte of interest in a biological sample (9) without the use of a chromatography unit, wherein the method comprises
A1) providing a clinical diagnostic system (100) according to any one of claims 1 to 10,
A) Automated preparation of the biological sample (9) comprising at least one analyte of interest using the sample preparation station (1) comprising a sample pre-treatment unit which comprises at least one unit or more than one units selected from the following group: magnetic bead handling unit, internal standard handling unit, supporting compound handling unit, pipetting unit, fluid transport unit, reaction container transporting mechanism unit, enrichment handling unit, solvent evaporation unit, derivatization unit,
B) Ion generation from the at least one analyte of interest using the ion generation station (2) being based on electrospray ionization,
C) Separation the ion or ions via their ion mobility using an electric field applied to one or more electrodes,
D) Detection and determining of at least one ion of the at least one analyte of interest using the ion detection station (4) based on mass spectrometry.

13. The method of claim 12, wherein an internal standard is added, preferably in step (A), wherein the internal standard is preferably isotopically labelled.

## Patentansprüche

1. System zur automatisierten klinischen Diagnose (100) zum Bestimmen der Anwesenheit oder des Spiegels von mindestens einem Analyten von Interesse in einer biologischen Probe, umfassend
- eine Probenvorbereitungsstation (1) für die automatisierte Vorbereitung von biologischen Proben (9), umfassend mindestens einen Analyten von Interesse,
- eine Ionenerzeugungsstation (2) zur Erzeugung von mindestens einem Ion oder von Ionen des mindestens einen Analyten von Interesse, wobei die Ionenerzeugungsstation (2) eine Einheit zur regelmäßigen Analytionenzufuhr umfasst, die eine Fließinjektion umfasst, welche Elektrospray-Ionisation verwendet;
- mindestens eine Selektivitätsverstärkerstation (3) oder mehr zur Verstärkung der Selektivität des mindestens einen Analyten von Interesse, wobei die mindestens eine Selektivitätsverstärkerstation (3) konfiguriert ist, um Ionen basierend auf der jeweiligen Ionengröße zu trennen, und eine Ionenmobilitätseinheit (31) zum Trennen von Ionen in Bezug auf ihre Ionenmobilität unter Verwendung von elektrischen und/oder Hochfrequenzfeldern (AC/DC und/oder RF) umfasst, die an eine oder mehrere Elektroden angelegt werden, wobei die Ionenmobilitätseinheit (31) keinen Stapel Leiterplatten umfasst,
- eine Ionendetektionsstation (4) zum Nachweis von mindestens einem Ion des mindestens einen Analyten von Interesse unter Verwendung von Massenspektrometrie und
- eine Datenverarbeitungsstation (5) zum Verarbeiten und/oder Auswerten von mindestens einem elektronischen Signal, das zumindest von dem Ionendetektionssystem empfangen wird,
wobei die Probenvorbereitungsstation (1) direkt mit der Ionenerzeugungsstation (2) verbunden ist, die direkt mit der mindestens einen Selektivitätsverstärkerstation (3) verbunden ist, die direkt mit der Ionendetektionsstation (4) verbunden ist,
wobei die Probenvorbereitungsstation eine Probenvorbehandlungseinheit umfasst, die mindestens eine Einheit oder mehr als eine Einheit umfasst, die aus der folgenden Gruppe ausgewählt sind: Magnetkügelchen-Handhabungseinheit, interner Standard-Handhabungseinheit, Trägerverbindung-Handhabungseinheit, Pipettiereinheit, Fluidtransporteinheit, Reaktionsbehälter-Transportmechanismuseinheit, Anreicherungs-Handhabungseinheit, Lösungsmittelverdampfungseinheit, Derivatisierungseinheit, und
wobei das System zur klinischen Diagnose (100) keine Chromatographieeinheit aufweist.

2. System zur klinischen Diagnose (100) nach Anspruch 1, wobei die biologische Probe (9) in einem Zufallsüberschussmodus vorbereitet wird.

3. System zur klinischen Diagnose (100) nach Anspruch 1 oder 2, wobei die biologische Probe (9) aus einer Patientenprobe erhalten wird, ausgewählt aus einer Gruppe, bestehend aus Serum-, Plasma-, Gewebe-, Liquor-, Atem-, Schweiß-, Sputum- und Vollblutprobe eines Individuums, wobei das Individuum vorzugsweise ein Säugetier, vorzugsweise ein Mensch, ist.

4. System zur klinischen Diagnose (100) nach einem der vorhergehenden Ansprüche, wobei der mindestens eine Analyt von Interesse eine Molmasse von kleiner als m/z = 2000, vorzugsweise kleiner als m/z = 1000, aufweist.

5. System zur klinischen Diagnose (100) nach einem der vorhergehenden Ansprüche, wobei die Probenvorbereitungsstation (1) zum Entfernen oder zumindest Verringern störender Matrixkomponenten in der biologischen Probe (9) und/oder zum Anreichern von Analyten von Interesse in der biologischen Probe (9) dient und mindestens eine der folgenden Einheiten oder Kombinationen davon umfasst: Probenabgabeeinheit, Probenanalyseeinheit.

6. System zur klinischen Diagnose (100) nach einem der vorhergehenden Ansprüche, wobei die Probenabgabeeinheit Probensammlung und -vorbereitung und/oder -transport umfasst, und/oder
wobei die Probenanalyseeinheit mindestens eine Einheit oder mehr als eine Einheit umfasst, ausgewählt aus der Gruppe: eine Fest-Flüssig-Träger-Analytanreicherungseinheit, Fest-Flüssig-Träger-Matrixabreicherungseinheit, Anreicherungs- oder -Matrixtrennungseinheit für gasförmige Proben, Festproben-auf-Festträger-Einheit.

7. System zur klinischen Diagnose (100) nach einem der vorhergehenden Ansprüche, wobei die Probenvorbereitungsstation (1) teilweise oder vollständig automatisiert ist.

8. System zur klinischen Diagnose (100) nach einem der vorhergehenden Ansprüche, wobei die Ionenerzeugungsstation (2) eine Einheit zur regelmäßigen Analytionenzufuhr (22) umfasst, wobei die Einheit zur unregelmäßigen Analytionenzufuhr (21) ausgewählt ist aus der Gruppe, bestehend aus Laserdesorptionsionisation (LDI, 211), dielektrischer Barriereflüssigkeitsströmung (212), Oberflächenplasmaionisation (213) und Kombinationen davon, wobei die Einheit zur regelmäßigen Analytionenzufuhr (22) ausgewählt ist aus der Gruppe, bestehend aus Fließinjektion (221), Papierspray (222), Elektronenionisation (EI, 223), matrixunterstützter Ionisation (MAI, 223), chemischer Ionisation (CI, 225), z. B. Protonentransferreaktion (PTR), radioaktiv unterstützter Ionisation (226), z. B. unter Verwendung von ⁶³Ni, und Kombinationen davon.

9. System zur klinischen Diagnose (100) nach einem der vorhergehenden Ansprüche, wobei die Selektivitätsverstärkerstation (3) die Ionenmobilitätseinheit (31) und mindestens eine Einheit umfasst, ausgewählt aus der folgenden Gruppe: Ionentransporteinheit (32), Ionenfragmentierungseinheit (33), wobei die Ionentransporteinheit (32) ausgewählt ist aus der Gruppe, bestehend aus Ionentrichtern (321), Stufenwelle (322), S-Linse (323), Ionenspiegel (324), Ionenflugrohr mit Differenzpotential (325), Ionenfalle (326), Multipol (327), z. B. Quadrupol, kontaktloses Ionentransportsystem (328), z. B. direkte Quellenkopplung oder beheizte Kapillare, und Magnetsektortransport (329), wobei die Ionenfragmentierungseinheit (33) ausgewählt ist aus der Gruppe, bestehend aus kollisionsinduzierter Dissoziation (CID, 331), Elektroneneinfangdissoziation (ECD, 332), Elektronentransferdissoziation (ETD, 333), Photonendissoziation (334), Ionenfalle (335), Orbitrap (336), Flugzeit (TOF, 337), Magnetsektor (338), z. B. Doppelfokussions-Sektorfeld (DFS) und Kombinationen davon.

10. System zur klinischen Diagnose (100) nach einem der vorhergehenden Ansprüche, wobei der mindestens eine Analyt von Interesse weniger als 10 Sekunden, vorzugsweise weniger als 9 Sekunden oder weniger als 8 Sekunden oder weniger als 7 Sekunden oder weniger als 6 Sekunden oder weniger als 5 Sekunden oder weniger als 4 Sekunden oder weniger als 3 Sekunden oder weniger als 2 Sekunden oder weniger als 1 Sekunde in dem System zur klinischen Diagnose (100) oder in dem Massenspektrometer oder in der Ionenmobilitätseinheit (31) vorhanden ist.

11. Verwendung des Systems zur klinischen Diagnose (100) nach einem der Ansprüche 1 bis 10 zum Bestimmen der Anwesenheit oder des Spiegels des mindestens einen Analyten von Interesse in der biologischen Probe (9).

12. Automatisiertes Verfahren zum Bestimmen der Anwesenheit oder des Spiegels von mindestens einem Analyten von Interesse in einer biologischen Probe (9) ohne die Verwendung einer Chromatographieeinheit, wobei das Verfahren Folgendes umfasst:
A1) Bereitstellen eines Systems zur klinischen Diagnose (100) nach einem der Ansprüche 1 bis 10,
A) Automatisierte Vorbereitung der biologischen Probe (9), umfassend mindestens einen Analyten von Interesse, unter Verwendung der Probenvorbereitungsstation (1), umfassend eine Probenvorbehandlungseinheit, die mindestens eine Einheit oder mehr als eine Einheit umfasst, ausgewählt aus der folgenden Gruppe: Magnetkügelchen-Handhabungseinheit, interner Standard-Handhabungseinheit, Trägerverbindung-Handhabungseinheit, Pipettiereinheit, Fluidtransporteinheit, Reaktionsbehälter-Transportmechanismuseinheit, Anreicherungs-Handhabungseinheit, Lösungsmittelverdampfungseinheit, Derivatisierungseinheit,
B) Ionenerzeugung aus dem mindestens einen Analyten von Interesse unter Verwendung der Ionenerzeugungsstation (2), die auf Elektrospray-Ionisation basiert,
C) Trennen des Ions oder der Ionen anhand ihrer Ionenmobilität unter Verwendung eines elektrischen Felds, das an eine oder mehrere Elektroden angelegt wird,
D) Detektion und Bestimmung von mindestens einem Ion des mindestens einen Analyten von Interesse unter Verwendung der Ionendetektionsstation (4) basierend auf Massenspektrometrie.

13. Verfahren nach Anspruch 12, wobei ein interner Standard zugegeben wird, vorzugsweise in Schritt (A), wobei der interne Standard vorzugsweise isotopenmarkiert ist.

## Revendications

1. Système de diagnostic clinique automatisé (100) pour la détermination de la présence ou le taux d'au moins un analyte d'intérêt dans un échantillon biologique comprenant
- une station de préparation d'échantillons (1) pour la préparation automatisée d'échantillons biologiques (9) comprenant au moins un analyte d'intérêt,
- une station de génération d'ions (2) pour la génération d'au moins un ion ou d'ions de l'au moins un analyte d'intérêt, la station de génération d'ions (2) comprenant une unité d'alimentation régulière en ions de l'analyte comprenant une injection en flux qui utilise une ionisation par électronébulisation ;
- au moins une station d'amélioration de la sélectivité (3) ou plusieurs pour l'amélioration de la sélectivité de l'au moins un analyte d'intérêt, l'au moins une station d'amélioration de la sélectivité (3) étant configurée pour séparer les ions sur la base de la taille respective des ions et comprenant une unité de mobilité ionique (31) pour la séparation des ions par rapport à leur mobilité ionique en utilisant des champs électriques et/ou radiofréquence (CA/CC et/ou RF) appliqués à une ou plusieurs électrodes, l'unité de mobilité ionique (31) étant exempte d'un empilement de cartes de circuits imprimés,
- une station de détection d'ions (4) pour la détermination d'au moins un ion de l'au moins un analyte d'intérêt, en utilisant la spectrométrie de masse, et
- une station de traitement de données (5) pour le traitement et/ou l'évaluation d'au moins un signal électronique reçu au moins du système de détection d'ions,
dans lequel la station de préparation d'échantillons (1) est directement connectée à la station de génération d'ions (2), qui est directement connectée à l'au moins une station d'amélioration de la sélectivité (3), qui est directement connectée à la station de détection d'ions (4),
dans lequel la station de préparation d'échantillons comprend une unité de prétraitement d'échantillons qui comprend au moins une unité ou plus d'une unité choisie dans le groupe suivant : une unité de manipulation de billes magnétiques, une unité de manipulation d'étalons internes, une unité de manipulation de composés de support, une unité de pipetage, une unité de transport de fluides, une unité de mécanisme de transport de récipients de réaction, une unité de gestion d'enrichissement, une unité d'évaporation de solvants, une unité de dérivation, et
dans lequel le système de diagnostic clinique (100) est exempt d'une unité de chromatographie.

2. Système de diagnostic clinique (100) selon la revendication 1, dans lequel l'échantillon biologique (9) est préparé selon un mode d'excès aléatoire.

3. Système de diagnostic clinique (100) selon la revendication 1 ou 2, dans lequel l'échantillon biologique (9) est obtenu d'un échantillon de patient, qui est choisi dans le groupe constitué par des échantillons de sérum, de plasma, de tissu, de liqueur, d'air expiré, de sueur, d'expectorations et de sang total d'un individu, de préférence dans lequel l'individu est un mammifère, de préférence un humain.

4. Système de diagnostic clinique (100) selon l'une quelconque des revendications précédentes, dans lequel l'au moins un analyte d'intérêt a une masse molaire inférieure à m/z = 2000, de préférence inférieure à m/z = 1000.

5. Système de diagnostic clinique (100) selon l'une quelconque des revendications précédentes, dans lequel la station de préparation d'échantillons (1) est destinée à éliminer ou au moins à réduire les composants matriciels interférents dans l'échantillon biologique (9) et/ou à enrichir les analytes d'intérêt dans l'échantillon biologique (9) et comprend au moins l'une des unités suivantes ou des combinaisons de celles-ci : une unité de distribution d'échantillons, une unité analytique d'échantillons.

6. Système de diagnostic clinique (100) selon l'une quelconque des revendications précédentes, dans lequel l'unité de distribution d'échantillons comprend la collecte et la préparation et/ou le transport d'échantillons, et/ou
dans lequel l'unité analytique d'échantillons comprend au moins une unité ou plus d'une unité choisie dans le groupe : une unité d'enrichissement d'analytes sur support solide-liquide, une unité d'appauvrissement matriciel sur support solide-liquide, une unité d'enrichissement ou de séparation matricielle d'échantillons gazeux, une unité d'échantillon solide sur support solide.

7. Système de diagnostic clinique (100) selon l'une quelconque des revendications précédentes, dans lequel la station de préparation d'échantillons (1) est en partie ou complètement automatisée.

8. Système de diagnostic clinique (100) selon l'une quelconque des revendications précédentes, dans lequel la station de génération d'ions (2) comprend une unité d'alimentation régulière en ions de l'analyte (22), dans lequel l'unité d'alimentation irrégulière en ions de l'analyte (21) est choisie dans le groupe constitué par une ionisation par désorption laser (IDL, 211), un flux de liquide à barrière diélectrique (212), une ionisation de surface par plasma (213) et des combinaisons de ceux-ci, dans lequel l'unité d'alimentation régulière en ions de l'analyte (22) est choisie dans le groupe constitué par une injection en flux (221), une pulvérisation sur papier (222), une ionisation électronique (IE, 223), une ionisation assistée par matrice (IAM, 223), une ionisation chimique (IC, 225), par exemple une réaction de transfert de protons (PTR), une ionisation sur support radioactif (226), par exemple en utilisant du ⁶³Ni et des combinaisons de ceux-ci.

9. Système de diagnostic clinique (100) selon l'une quelconque des revendications précédentes, dans lequel la station d'amélioration de la sélectivité (3) comprend l'unité de mobilité ionique (31) et au moins une unité choisie dans le groupe suivant : une unité de transport d'ions (32), une unité de fragmentation d'ions (33), dans lequel l'unité de transport d'ions (32) est choisie dans le groupe constitué par des entonnoirs à ions (321), une onde en escalier (322), une lentille en S (323), un miroir à ions (324), un tube de lutte ionique par potentiel différentiel (325), un piège à ions (326), un multipôle (327), par exemple un quadripôle, un système de transport d'ions sans contact (328), par exemple un couplage direct de sources ou un capillaire chauffé, et un transport par secteur magnétique (329), dans lequel l'unité de fragmentation d'ions (33) est choisie dans le groupe constitué par une dissociation induite par collision (DIC, 331), une dissociation par capture d'électrons (DCE, 332), une dissociation par transfert d'électrons (DTE, 333), une dissociation photonique (334), un piège à ions (335), un Orbitrap (336), un temps de vol (TOF, 337), un secteur magnétique (338), par exemple un champ sectoriel à double focalisation (DFS) et des combinaisons de ceux-ci.

10. Système de diagnostic clinique (100) selon l'une quelconque des revendications précédentes, dans lequel l'au moins un analyte d'intérêt est présent dans ledit système de diagnostic clinique (100) ou dans le spectromètre de masse ou dans l'unité de mobilité ionique (31) pendant moins de 10 secondes, de préférence moins de 9 secondes ou moins de 8 secondes ou moins de 7 secondes ou moins de 6 secondes ou moins de 5 secondes ou moins de 4 secondes ou moins de 3 secondes ou moins de 2 secondes ou moins de 1 seconde.

11. Utilisation du système de diagnostic clinique (100) selon l'une quelconque des revendications 1 à 10 pour déterminer la présence ou le taux de l'au moins un analyte d'intérêt dans l'échantillon biologique (9).

12. Procédé automatisé pour la détermination de la présence ou du taux d'au moins un analyte d'intérêt dans un échantillon biologique (9) sans l'utilisation d'une unité de chromatographie, dans lequel le procédé comprend
A1) la fourniture d'un système de diagnostic clinique (100) selon l'une quelconque des revendications 1 à 10,
A) la préparation automatisée de l'échantillon biologique (9) comprenant au moins un analyte d'intérêt en utilisant la station de préparation d'échantillons (1) comprenant une unité de prétraitement d'échantillons qui comprend au moins une unité ou plus d'une unité choisie dans le groupe suivant : une unité de manipulation de billes magnétiques, une unité de manipulation d'étalons internes, une unité de manipulation de composés de support, une unité de pipetage, une unité de transport de fluides, une unité de mécanisme de transport de récipients de réaction, une unité de gestion d'enrichissement, une unité d'évaporation de solvants, une unité de dérivation,
B) la génération d'ions à partir de l'au moins un analyte d'intérêt en utilisant la station de génération d'ions (2) qui est basée sur une ionisation par électronébulisation,
C) la séparation de l'ion ou des ions par l'intermédiaire de leur mobilité ionique en utilisant un champ électrique appliqué à une ou plusieurs électrodes,
D) la détection et la détermination d'au moins un ion de l'au moins un analyte d'intérêt en utilisant la station de détection d'ions (4) sur la base de la spectrométrie de masse.

13. Procédé selon la revendication 12, dans lequel un étalon interne est ajouté, de préférence à l'étape (A), dans lequel l'étalon interne est de préférence marqué de manière isotopique.
